(19) Europäisches Patentamt
European Patent Office
Office européen des brevets

(11) **EP 4 708 303 A1**

(12) **EUROPEAN PATENT APPLICATION**

(43) Date of publication:
**11.03.2026 Bulletin 2026/11**

(21) Application number: **25199201.2**

(22) Date of filing: **29.08.2025**

(51) International Patent Classification (IPC):
**G16C 20/20** (2019.01)   **G16C 20/70** (2019.01)
**G16B 40/10** (2019.01)

(52) Cooperative Patent Classification (CPC):
**G16C 20/70; G16C 20/20;** G16B 40/10

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **03.09.2024 US 202463689981 P**

(71) Applicants:
• **Thermo Electron SAS**
  **91140 Villebon-sur-Yvette (FR)**

• **HighChem s.r.o.**
  **821 09 Bratislava (SK)**

(72) Inventors:
• **LACOUT, Fabrice**
  **Salon de Provence (FR)**
• **ZSEMLYE, Gábor**
  **Samorín (SK)**

(74) Representative: **Boult Wade Tennant LLP**
**Salisbury Square House**
**8 Salisbury Square**
**London EC4Y 8AP (GB)**

(54) **CONSTRUCTING IN SILICO MASS SPECTRA OF COMPOUNDS**

(57)    Disclosed herein are scientific instrument support systems, as well as related methods, computing devices, and computer-readable media that can be employed to construct *in silico* mass spectra of compounds. In various embodiments, a system can comprise a processor that can execute computer-executable components stored in a non-transitory computer-readable memory, wherein the computer-executable components can comprise a matrix computation component that can compute a Markov transition matrix and a mass spectrum component that can construct a mass spectrum for a molecule based on the Markov transition matrix.

FIG. 17

EP 4 708 303 A1

**Description**

CROSS-REFERENCE TO RELATED APPLICATIONS

This application claims priority to and the benefit of Provisional Patent Application No. 63,689/981 filed on September 03, 2024, entitled "CONSTRUCTING IN SILICO MASS SPECTRA OF COMPOUNDS." The entireties of the aforementioned application are incorporated by reference herein.

BACKGROUND

Mass spectral libraries of known compounds can be employed to identify unknown compounds. However, such libraries can be limited by the number of known compounds, and identification of certain unknown compounds can be challenging.

SUMMARY

The following presents a summary to provide a basic understanding of one or more embodiments described herein. This summary is not intended to identify key or critical elements, delineate scope of particular embodiments or scope of claims. Its sole purpose is to present concepts in a simplified form as a prelude to the more detailed description that is presented later. In one or more embodiments described herein, systems, computer-implemented methods, apparatus and/or computer program products that can be employed to construct silico mass spectra of compounds are discussed.

According to an embodiment, a system is provided. The system can comprise a processor that can execute computer-executable components stored in a non-transitory computer-readable memory, wherein the computer-executable components can comprise a matrix computation component that can compute a Markov transition matrix. The computer-executable components can further comprise a mass spectrum component that can construct a mass spectrum for a molecule based on the Markov transition matrix.

According to an embodiment, a computer-implemented method is provided. The computer-implemented method can comprise computing, by a device operatively coupled to a processor, a Markov transition matrix. The computer-implemented method can further comprise constructing, by the device, a mass spectrum for a molecule based on the Markov transition matrix.

According to an embodiment, a computer program product is provided. The computer program product can comprise a non-transitory computer-readable memory having program instructions embodied therewith. The program instructions can be executable by a processor to cause the processor to compute a Markov transition matrix. The program instructions can be further executable by a processor to cause the processor to construct a mass spectrum for a molecule based on the Markov transition matrix.

BRIEF DESCRIPTION OF THE DRAWINGS

Embodiments will be readily understood by the following detailed description in conjunction with the accompanying drawings. To facilitate this description, like reference numerals designate like structural elements. Embodiments are illustrated by way of example, not by way of limitation, in the figures of the accompanying drawings.

FIG. 1 illustrates a block diagram of an example, non-limiting scientific instrument support module, in accordance with various embodiments described herein.

FIG. 2 illustrates a flow diagram of an example, non-limiting computer-implemented method, in accordance with various embodiments described herein.

FIG. 3 illustrates a block diagram of an example, non-limiting system that can generate *in silico* mass spectra of compounds, in accordance with various embodiments described herein.

FIG. 4 illustrates another block diagram of an example, non-limiting system that can generate *in silico* mass spectra of compounds, in accordance with various embodiments described herein.

FIG. 5 illustrates yet another block diagram of an example, non-limiting system that can generate *in silico* mass spectra of compounds, in accordance with various embodiments described herein.

FIG. 6 illustrates a block diagram of an example, non-limiting system that can employ an *in silico* mass spectral database to identify unknown compounds, in accordance with various embodiments described herein.

FIG. 7 illustrates a diagram of an example, non-limiting fragmentation graph that can be generated via a simulated fragmentation of an ionized molecule, in accordance with various embodiments described herein.

FIGS. 8A - 8C illustrate a diagram of an example, non-limiting fragmentation graph with reaction probabilities that can be generated via a machine learning software, in accordance with various embodiments described herein.

FIG. 9 illustrates a diagram of an example, non-limiting fragmentation graph with reaction probabilities and self-going edges that can be generated via a machine learning software, in accordance with various embodiments described herein.

FIG. 10 illustrates a diagram of an example, non-limiting mass spectrum, in accordance with various embodiments described herein.

FIG. 11 illustrates a diagram of an example, non-limiting fragmentation graph showing a random walk process, in accordance with various embodiments described herein.

FIG. 12 illustrates an example, non-limiting histogram and an example, non-limiting mass spectrum based on a random walk process involving a Markov transition matrix, in accordance with various embodiments described herein.

FIG. 13A illustrates example, non-limiting molecules, in accordance with various embodiments described herein.

FIG. 13B illustrates example, non-limiting mass spectra corresponding to the molecules illustrated in FIG. 13A, in accordance with various embodiments described herein.

FIG. 14 illustrates example, non-limiting molecules, in accordance with various embodiments described herein.

FIG. 15A illustrates example, non-limiting mass spectra generated by a mass spectrometer, in accordance with various embodiments described herein.

FIG. 15B illustrates example, non-limiting mass spectra generated by a software, in accordance with various embodiments described herein.

FIG. 16 illustrate example, non-limiting barcode graphs, in accordance with various embodiments described herein.

FIG. 17 illustrates a flow diagram of an example, non-limiting method that can construct *in silico* mass spectra for compounds, in accordance with various embodiments described herein.

FIG. 18 is a block diagram of an example, non-limiting graphical user interface (GUI) that can be used in the performance of some or all of the methods or techniques disclosed herein, in accordance with various embodiments described herein.

FIG. 19 is a block diagram of an example, non-limiting computing device that can perform some or all of the methods or techniques disclosed herein, in accordance with various embodiments described herein.

FIG. 20 is a block diagram of an example, non-limiting scientific instrument support system in which some or all of the methods or techniques disclosed herein may be performed, in accordance with various embodiments described herein.

FIG. 21 illustrates a block diagram of an example, non-limiting operating environment in which one or more embodiments described herein can be facilitated.

FIG. 22 illustrates an example networking environment operable to execute various implementations described herein.

## DETAILED DESCRIPTION

The following detailed description is merely illustrative and is not intended to limit embodiments and/or application or uses of embodiments. Furthermore, there is no intention to be bound by any expressed or implied information presented in the preceding Background or Summary sections, or in the Detailed Description section.

One or more embodiments are now described with reference to the drawings, wherein like referenced numerals are used to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

DEFINITIONS:

*In silico: "In silico"* refers to experiments conducted, or to data generated to study various phenomena via computer-based simulations, modeling or analysis as opposed to direct physical experimentation. For example, an *in silico* fragmentation of a molecule can refer to a computer simulated fragmentation of the molecule.

Markov process: The Markov process is a model that describes a sequence of possible events, wherein the probability of each event depends on a state associated with a previous event.

Disclosed herein are scientific instrument support systems, as well as related methods, computing devices, and computer-readable media. For example, in some embodiments, a system can comprise a processor that can execute computer-executable components stored in a non-transitory computer-readable memory, wherein the computer-executable components can comprise a matrix computation component that can compute a Markov transition matrix. The computer-executable components can further comprise a mass spectrum component that can construct a mass spectrum for a molecule based on the Markov transition matrix.

The scientific instrument support embodiments disclosed herein may achieve improved performance relative to conventional approaches. For example, mass spectral (MS) databases and libraries such as, for example, the National Institute of Standards and Technology (NIST), MassBank, myLibrary or mzCloud™ for known chemical compounds currently play an irreplaceable role in the identification of unknown compounds. However, the information contained in such libraries is limited relative to the number of chemical compounds that are known to exist, which limits the number of unknown compounds that can be identified via such databases and libraries. In silico libraries can comprise in silico (e.g., synthetic) mass spectra of chemical compounds, and have the potential to provide unlimited search capabilities. However, conventional approaches for generating in silico mass spectra can be computationally intensive, consume large amounts of memory and may not be supported by graphics processing unit (GPUs).

On the contrary, various embodiments of the present disclosure can more efficiently construct in silico mass spectra of chemical compounds such as small molecules. For example, various ones of the embodiments disclosed herein may improve upon conventional approaches to achieve the technical advantages of fewer computations, shorter computing times and smaller memory consumption as compared to the conventional approaches by employing a matrix-based approach that is supported by GPUs. Such technical advantages are not achievable by routine and conventional approaches, and all users of systems including such embodiments may benefit from these advantages (e.g., by assisting the user in the performance of a technical task, such as identification of chemical compounds, by means of a guided human-machine interaction process). The technical features of the embodiments disclosed herein are thus decidedly unconventional in the field of mass spectrometry, as are the combinations of the features of the embodiments disclosed herein.

As discussed further herein, various aspects of the embodiments disclosed herein may improve the functionality of a computer itself. For example, the matrix-based approach employed herein to generate in silico mass spectra of chemical compounds can represent fragmentation graphs of molecules as sparse matrices, and operations on sparse matrices are supported by many existing computational libraries such as PyTorch®, even on GPUs. As a result, the embodiments disclosed herein can enable computers to generate in silico mass spectra of chemical compounds in a less computationally intensive manner as compared to conventional approaches. The computational and user interface features disclosed herein do not only involve the collection and comparison of information, but apply new analytical and technical techniques to change and/or augment the operations of systems involved in mass spectrometry and compound identification. The present disclosure thus introduces functionality that neither a conventional computing device, nor a human, could perform. Further, the embodiments disclosed herein provide improvements to scientific instrument technology (e.g., improvements in the computer technology supporting such scientific instruments, among other improvements).

More specifically, embodiments described herein include systems (e.g., scientific instrument support systems), computer-implemented methods, and computer program products that can efficiently construct in silico mass spectra of chemical compounds via a matrix-based approach. Accordingly, in various embodiments, techniques to generate an output comprising an in silico mass spectrum based on an input comprising information about a chemical compound are presented. As such, the techniques presented herein can be employed to predict in silico mass spectra of several chemical compounds, wherein such in silico mass spectra can be employed in mass spectral databases of small molecules to identify unknown chemical compounds.

For example, in one or more embodiments, a set of potential ionized fragments of an ionized molecule can be calculated based on information describing the ionized molecule by simulating the manner in which the ionized molecule can be expected to fragment (break down) into fragment ions in a mass spectrometer. Such a simulation can be performed via a software such as, for example, SledgeHammer. For a given input molecule and ionization method, SledgeHammer can generate a fragmentation graph. For example, upon receiving information about an input molecule (i.e., a molecule to be analyzed, an unknown chemical compound, etc.), SledgeHammer can simulate an ionization of the input molecule to generate an ionized molecule, followed by simulating a fragmentation of the ionized molecule. The ionized molecule thus generated can be an in silico molecule. SledgeHammer can represent the fragmentation via a fragmentation graph. The fragmentation graph can be a directed graph of ion fragments (products) that can result from a fragmentation of the ionized molecule, wherein each node can represent an ion fragment and each edge connecting two nodes can represent a chemical reaction that indicates the child ion fragments that can be generated from a parent ion fragment. Since each ion fragment can fragment in several ways, each parent ion fragment can result in several child ion fragments. Neutral losses are omitted from the fragmentation graph. As such, the input molecule can be the root/parent node in the fragmentation graph and other nodes in the fragmentation graph can represent potential ion fragments resulting from the fragmentation of the input molecule. The only child node of the input molecule can be the ionized molecule, and the other child nodes in the fragmentation graph can be ion fragments resulting from fragmentation of the ionized molecule.

In one or more embodiments, the fragmentation graph can be employed to calculate reaction probabilities (probabilities of reactions; can also be referred to as edge probabilities in a fragmentation graph) for each chemical reaction occurring in the fragmentation graph. For example, the probability of each chemical reaction yielding a specific ion fragment can be calculated. The reaction probabilities can be calculated via existing software that can employ machine learning algorithms, and the output of the software can be another fragmentation graph with numeric values representing the reaction probabilities. The new fragmentation graph thus generated can also comprise self-going edges that represent chemical reactions that do not occur. For example, a pre-processing algorithm can extend the fragmentation graph generated in the previous step by adding self-going edges for nodes. A self-going edge represents an ion fragment that does not further fragment into additional fragments. For each set of outgoing reaction probabilities associated with a parent node, including the reaction probabilities of any self-going nodes, the sum of reaction probabilities should equal to 1.

The reaction probabilities can be employed to compute mass spectra for molecules, wherein the mass spectra can be *in silico* mass spectra. For example, in one or more embodiments, the reaction probabilities generated in the previous step can be employed to calculate *in silico* ion fragments and an *in silico* mass spectrum for the input molecule. First, respective reaction probabilities can be transformed into respective fragment probabilities (probabilities of fragments). A fragment probability represents the probability of an ion fragment to occur. The calculation to transform the reaction probabilities into fragment probabilities can be performed by employing Markov chains and a Markov transition matrix, wherein the fragmentation can be modeled via a Markov process and the fragmentation graph can be considered a Markov chain. The Markov process is a model that describes a sequence of possible events, wherein the probability of each event depends on a state associated with a previous event. For example, an unfragmented ionized molecule can represent an initial state of fragmentation of the system of the input molecule, and the fragmentation graph with the reaction probabilities can define the state transition of the unfragmented ionized molecule, wherein each ion fragment can produce one or more additional ion fragments, including itself (i.e., no further fragmentation), with respective probabilities. Then, the fragmented state of the system of the input molecule can be defined by the fragment probabilities of ion fragments to occur (i.e., current probability of ion fragments to occur).

More specifically, in one or more embodiments, a software can be employed to compute *in silico* mass spectra for molecules. For example, in one or more embodiments, the software can comprise a matrix computation component that can compute a Markov transition matrix based on respective reaction probabilities associated with a fragmentation of a molecule into a plurality of ion fragments. The Markov transition matrix can represent a transition of the molecule from an unfragmented state to a fragmented state. The software can further comprise a mass spectrum component that can generate a mass spectrum for the molecule based on reaction probabilities associated with fragmentation of the molecule. To generate the mass spectrum, the software can employ a probability determination component that can transform the respective reaction probabilities into respective fragment probabilities associated with the fragmentation of the molecule based on the Markov transition matrix. Herein, the molecule can represent an input molecule such as previously discussed, wherein information about the input molecule can be processed by another software such as SledgeHammer to ionize the molecule and generate a fragmentation graph showing the fragmentation of the ionized molecule into ion fragments, followed by further processing by machine learning algorithms to generate reaction probabilities based on the fragmentation graph. Thereafter, the respective reaction probabilities can be accessed by the probability determination component and transformed into the respective fragment probabilities.

In one or more embodiments, the probability determination component can transform the respective reaction probabilities into the respective fragmentation probabilities by employing the Markov transition matrix in a random walk process to generate a desired state within a fragmentation of the molecule. By doing so, the probability determination component can model the fragmentation as a Markov process. In one or more embodiments, the software can further comprise a peak intensity computation component that can compute a sum of fragment probabilities of respective ion fragments having identical masses. For example, the peak intensity for each ion fragment can be the sum of fragment probabilities of all ion fragments having mass identical to that of the ion fragment. The different intensities thus generated by the peak intensity computation component can be the mass spectrum that can be displayed to an end entity (e.g., hardware, software, machine, artificial intelligence (AI), neural network and/or user) on a suitable device. For example, in one or more embodiments, the software can further comprise a display component that can display the mass spectrum at a device such as a scientific instrument, a desktop computer, a laptop, a smartphone, etc. via a GUI. In some embodiments, the display component can also display the sum of fragment probabilities as a peak on a spectrogram. In this regard, in some implementations, the probability determination component, peak intensity computation component, and display component can be comprised in the mass spectrum component, whereas in other embodiments, the probability determination component, peak intensity computation component, and display component can be comprised in the software as components separate from the mass spectrum component.

In one or more embodiments, the software can further comprise a spectral database component that can generate a spectral database based on the mass spectrum, wherein the spectral database can be employable for compound identification. For example, the spectral database component can generate an *in silico* mass spectral database of the *in silico* mass spectra generated by the mass spectrum component for different molecules. In an implementation, the *in silico* mass spectral database can be stored in a memory and made available to an end entity (e.g., hardware, software, machine, AI, neural network and/or user) via a software application at a device such as a scientific instrument, a desktop computer, a laptop, a smartphone, etc. In another implementation, the *in silico* mass spectral library can be stored in a memory and made available to the end entity via a cloud environment. The end entity can employ the *in silico* mass spectral library for identification of unknown compounds. The embodiments disclosed herein thus provide improvements to mass spectrometry technology and chemical compound identification (e.g., improvements in the computer technology supporting mass spectrometry and chemical compound identification, among other improvements).

Currently, to identify compounds, a sample of the compound is measured, a mass spectrum is generated, and the mass spectrum is searched in spectral databases or libraries (e.g., mzCloud™ or any other). But if the measured spectrum is not available in a database or library, the compound cannot be identified. By employing embodiments of the present disclosure, a database of *in silico* mass spectra can be generated. When an end entity has some suspicions about what an unknown compound could be, an experimental mass spectrum of that compound can be generated with a mass spectrometer, and the experimental mass spectrum can be compared to *in silico* mass spectra comprised in the database.

Accordingly, the embodiments of the present disclosure may serve any of a number of technical purposes, such as generating *in silico* mass spectra of compounds such as small molecules; identifying unknown compounds (e.g., suspected compounds); generating *in silico* mass spectral libraries; or reducing the computational time and resources involved in the generation of *in silico* mass spectral libraries. In particular, the present disclosure provides technical solutions to technical problems, including but not limited to chemical compound identification based on *in silico* mass spectra.

In the following detailed description, reference is made to the accompanying drawings that form a part hereof wherein like numerals designate like parts throughout, and in which is shown, by way of illustration, embodiments that may be practiced. It is to be understood that other embodiments may be utilized, and structural or logical changes may be made, without departing from the scope of the present disclosure. Therefore, the following detailed description is not to be taken in a limiting sense.

Various operations may be described as multiple discrete actions or operations in turn, in a manner that is most helpful in understanding the subject matter disclosed herein. However, the order of description should not be construed as to imply that these operations are necessarily order dependent. In particular, these operations may not be performed in the order of presentation. Operations described may be performed in a different order from the described embodiment. Various additional operations may be performed, and/or described operations may be omitted in additional embodiments.

For the purposes of the present disclosure, the phrases "A and/or B" and "A or B" mean (A), (B), or (A and B). For the purposes of the present disclosure, the phrases "A, B, and/or C" and "A, B, or C" mean (A), (B), (C), (A and B), (A and C), (B and C), or (A, B, and C). Although some elements may be referred to in the singular (e.g., "a processing device"), any appropriate elements may be represented by multiple instances of that element, and vice versa. For example, a set of operations described as performed by a processing device may be implemented with different ones of the operations performed by different processing devices. As used herein, the phrase "based on" should be understood to mean "based at least in part on," unless otherwise specified.

The description uses the phrases "an embodiment," "various embodiments," and "some embodiments," each of which may refer to one or more of the same or different embodiments. Furthermore, the terms "comprising," "including," "having," and the like, as used with respect to embodiments of the present disclosure, are synonymous. When used to describe a range of dimensions, the phrase "between X and Y" represents a range that includes X and Y. As used herein, an "apparatus" may refer to any individual device, collection of devices, part of a device, or collections of parts of devices. The drawings are not necessarily to scale.

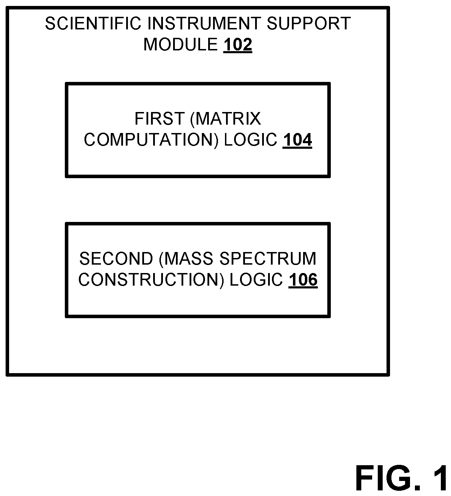

FIG. 1 illustrates a block diagram of an example, non-limiting scientific instrument support module 102, in accordance with various embodiments described herein.

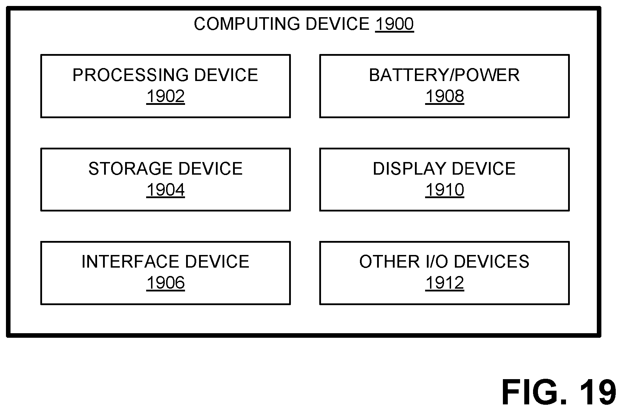

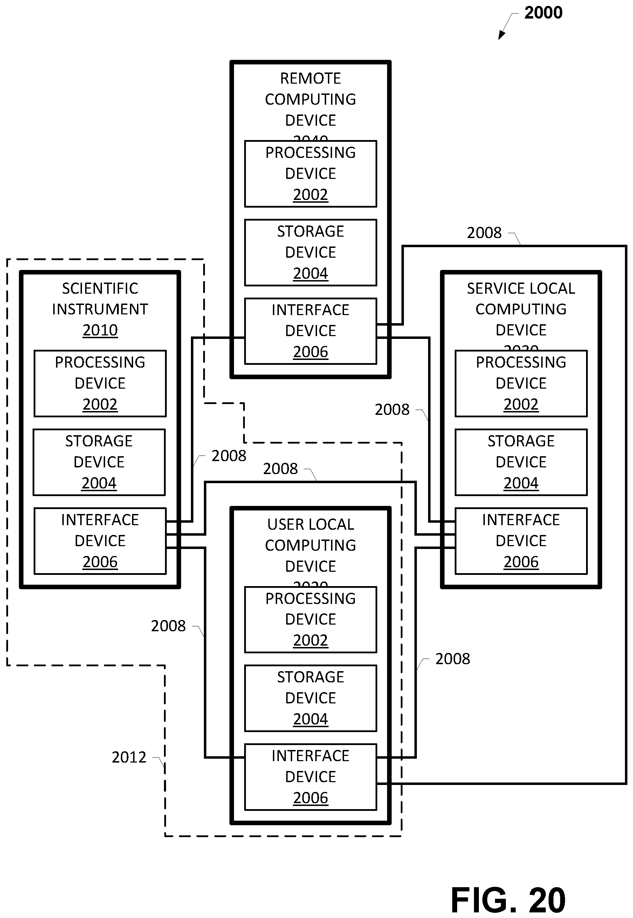

The scientific instrument support module 102 may be implemented by circuitry (e.g., including electrical and/or optical components), such as a programmed computing device. The logic of the scientific instrument support module 102 may be included in a single computing device, or may be distributed across multiple computing devices that are in communication with each other as appropriate. Examples of computing devices that may, singly or in combination, implement the scientific instrument support module 102 are discussed herein with reference to the computing device 1900 of FIG. 19, and examples of systems of interconnected computing devices, in which the scientific instrument support module 102 may be implemented across one or more of the computing devices, is discussed herein with reference to the scientific instrument support system 2000 of FIG. 20.

The scientific instrument support module 102 may include first logic 104 and second logic 106. As used herein, the term "logic" may include an apparatus that is to perform a set of operations associated with the logic. For example, any of the logic elements included in the scientific instrument support module 102 may be implemented by one or more computing devices programmed with instructions to cause one or more processing devices of the computing devices to perform the associated set of operations. In a particular embodiment, a logic element may include one or more non-transitory computer-readable media having instructions thereon that, when executed by one or more processing devices of one or more computing devices, cause the one or more computing devices to perform the associated set of operations. As used herein, the term "module" may refer to a collection of one or more logic elements that, together, perform a function associated with the module. Different ones of the logic elements in a module may take the same form or may take different forms. For example, some logic in a module may be implemented by a programmed general-purpose processing device, while other logic in a module may be implemented by an application-specific integrated circuit (ASIC). In another example, different ones of the logic elements in a module may be associated with different sets of instructions executed by one or more processing devices. A module may not include all of the logic elements depicted in the associated drawing; for example, a module may include a subset of the logic elements depicted in the associated drawing when that module is to perform a subset of the operations discussed herein with reference to that module.

In one or more embodiments, the first logic 104 can generate a Markov transition matrix, wherein the Markov transition matrix can represent a transition of a molecule from an unfragmented state to a fragmented state. First logic 104 can compute the Markov transition matrix based on respective reaction probabilities associated with fragmentation of the molecule, wherein a reaction probability of the respective reaction probabilities refers to the probability of a single chemical reaction occurring during the fragmentation. The reaction probabilities employed by first logic 104 to compute the Markov transition matrix can be defined in a fragmentation graph associated with the fragmentation and accessible to first logic 104. Each element comprised in the Markov transition matrix can represent a reaction probability corresponding to the fragmentation.

Recall that the fragmentation graph comprises nodes that represent ion fragments generated during the fragmentation of an ionized molecule and edges that represent chemical reactions that generate the ion fragments. As such, the fragmentation graph can comprise certain nodes that are not connected by edges. For example, a parent ion fragment can generate two child ion fragments, but the individual child ions fragments may not have a chemical reaction occurring between them. Thus, the Markov transition matrix can comprise elements with both zero, and non-zero values, wherein an elements with a zero value can respectively correspond to a chemical reactions that has a zero probability of occurrence, and an elements with a non-zero value can respectively correspond to a chemical reaction that has a probability of occurrence greater than zero. In this regard, the Markov transition matrix can be described as a graph adjacency matrix.

In one or more embodiments, second logic 106 can generate a mass spectrum based on the Markov transition matrix. For example, second logic 106 can employ the Markov transition matrix in a random walk process with a predefined number of steps to transform the respective reaction probabilities into respective fragment probabilities. A fragment probability of the respective fragment probabilities refers to the probability of an ion fragment being generated during the fragmentation. For example, for a pair of parent and child ion fragments, the reaction probability can be the probability of the chemical reaction that generates the child ion fragment occurring, and the fragment probability can be the probability of the child ion fragment being generated. More generally, a reaction probability refers to the probability of a process (i.e., a chemical reaction) and a fragmentation probability refers to the probability of a product (i.e., an ion fragment). To employ the Markov transition matrix in the random walk process, second logic 106 can represent a state of the fragmentation as a vector having a size equal to the number of ion fragments at that state, such that multiplying the Markov transition matrix with a vector representing any state in the fragmentation can generate another vector that can represent a subsequent state in the fragmentation.

For example, the state of the fragmentation when the molecule is an unfragmented ionized molecule can be represented as a column vector (e.g., by second logic 106) with a single non-zero element, and multiplying the Markov transition matrix with the vector once (i.e., one step of the random walk process) can generate a vector that can represent the immediate next step in the fragmentation. Similarly, multiplying the Markov transition matrix with the vector three times (i.e., three steps of the random walk process) can generate a vector that can represent the fourth step in the fragmentation, and so on. Each element in the resultant vector can represent a fragment probability for an ion fragment occurring at that state in the fragmentation, and second logic 106 can compute a sum of fragment probabilities of respective ion fragments having identical masses. For an ion fragment with a particular mass, the corresponding sum of fragment probabilities can represent its intensity. The intensities thus generated for ion fragments with unique masses can comprise the *in silico* mass spectrum. In one or more embodiments, second logic 106 can display the sum of fragment probabilities as a peak on a spectrogram. In one or more embodiments, such *in silico* mass spectra can be employed to generate mass spectral databases that can be further employed for identification of compounds (e.g., chemical compound such as small molecules, etc.).

Employing the Markov transition matrix to generate *in silico* mass spectra can be more computationally efficient than other techniques that tend to consume more computational time and memory. Thus, the scientific instrument support module 102 can enable efficient construction of in silico mass spectra.

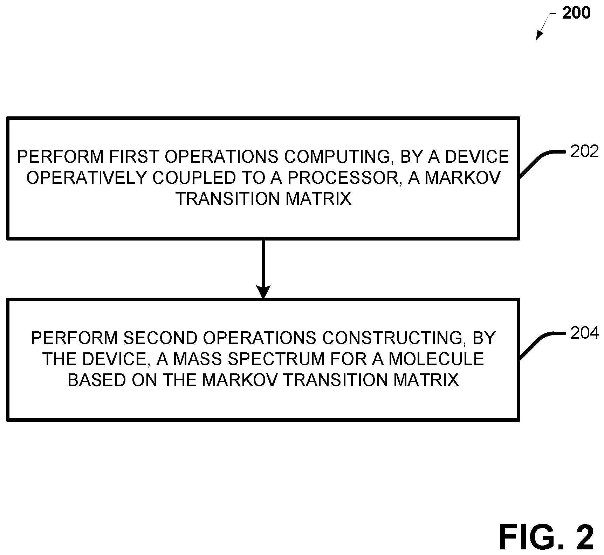

FIG. 2 illustrates a flow diagram of an example, non-limiting computer-implemented method 200, in accordance with various embodiments described herein. Repetitive description of like elements and/or processes employed in respective embodiments is omitted for sake of brevity.

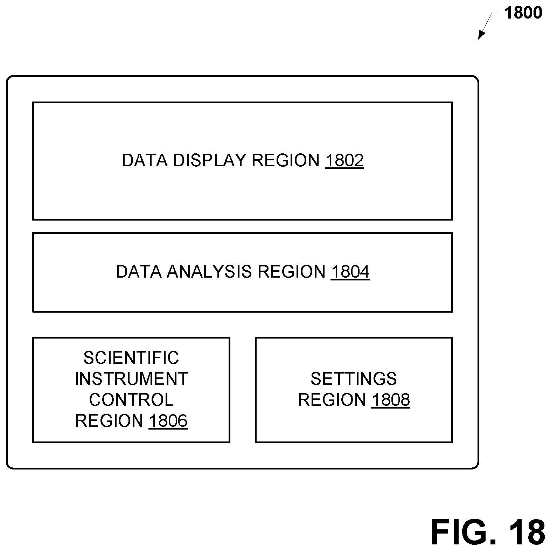

Although the operations of computer-implemented method 200 may be illustrated with reference to particular embodiments disclosed herein (e.g., the scientific instrument support modules 102 discussed herein with reference to FIG. 1, the GUI 1800 discussed herein with reference to FIG. 18, the computing devices 1900 discussed herein with reference to FIG. 19, and/or the scientific instrument support system 2000 discussed herein with reference to FIG. 20), non-limiting computer-implemented method 200 may be used in any suitable setting to perform any suitable support operations. Operations are illustrated once each and in a particular order in FIG. 2, but the operations may be reordered and/or repeated as desired and appropriate (e.g., different operations performed may be performed in parallel, as suitable).

At 202, first operations can be performed. For example, in various embodiments, first logic 104 of scientific instrument support module 102 can perform first operations computing, by a device operatively coupled to a processor, a Markov transition matrix.

At 204, second operations may be performed. For example, in various embodiments, second logic 106 of scientific instrument support module 102 can perform second operations constructing, by the device, a mass spectrum for a molecule based on the Markov transition matrix.

The scientific instrument support methods disclosed herein may include interactions with a human user (e.g., via the user local computing device 2020 discussed herein with reference to FIG. 20). These interactions may include providing information to the user (e.g., information regarding the operation of a scientific instrument such as the scientific instrument 2010 of FIG. 20, information regarding a sample being analyzed or other test or measurement performed by a scientific instrument, information retrieved from a local or remote database, or other information) or providing an option for a user to input commands (e.g., to control the operation of a scientific instrument such as the scientific instrument 2010 of FIG. 20, or to control the analysis of data generated by a scientific instrument), queries (e.g., to a local or remote database), or other information. In some embodiments, these interactions may be performed through a graphical user interface (GUI) that includes a visual display on a display device (e.g., the display device 1910 discussed herein with reference to FIG. 19) that provides outputs to the user and/or prompts the user to provide inputs (e.g., via one or more input devices, such as a keyboard, mouse, trackpad, or touchscreen, included in the other I/O devices 1912 discussed herein with reference to FIG. 19). The scientific instrument support systems disclosed herein may include any suitable GUIs for interaction with a user.

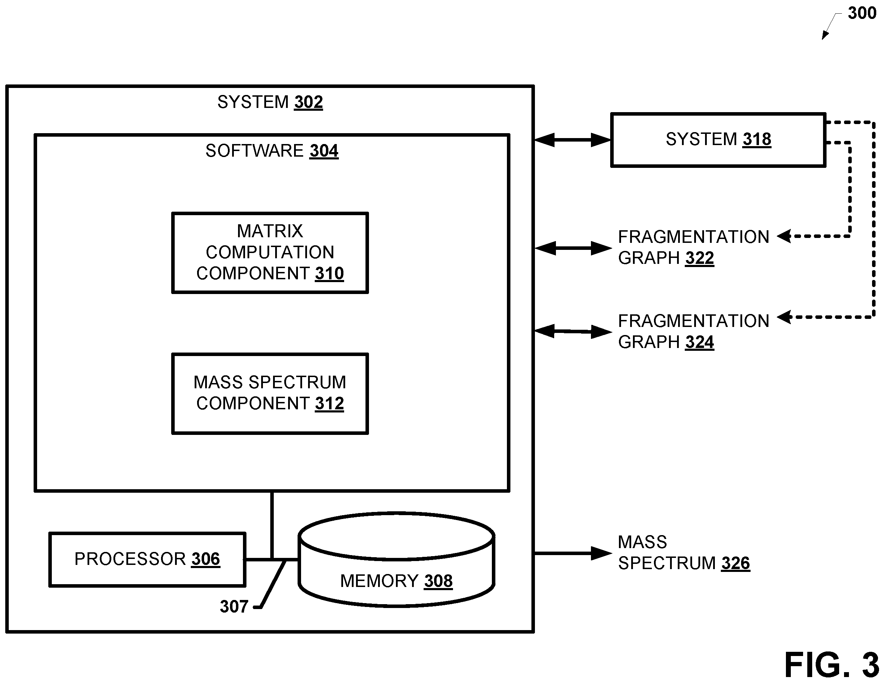

FIG. 3 illustrates a block diagram of an example, non-limiting system 300 that can generate in silico mass spectra of compounds, in accordance with various embodiments described herein. Repetitive description of like elements and/or processes employed in respective embodiments is omitted for sake of brevity.

Non-limiting system 300 and/or the components of non-limiting system 300 can be employed to use hardware and/or software to solve problems that are highly technical in nature (e.g., related to mass spectrometry, *in silico* mass spectra, compound identification, etc.), that are not abstract and that cannot be performed as a set of mental acts by a human. Further, some of the processes performed may be performed by specialized computers for carrying out defined tasks related to computation of *in silico* mass spectra. Non-limiting system 300 and/or components of non-limiting system 300 can be employed to solve new problems that arise through advancements in technologies mentioned above and/or the like. Non-limiting system 300 can provide technical improvements to systems employed in the field of mass spectrometry by reducing the computational load, increasing the computational speed and reducing the memory consumption involved in the generation of *in silico* mass spectra.

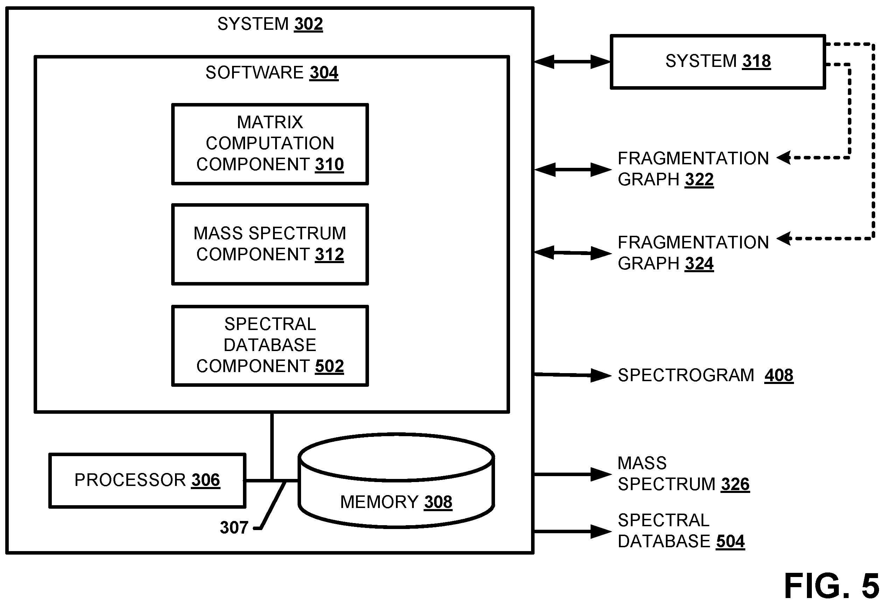

Non-limiting system 300 can be employed to generate *in silico* mass spectra of compounds that can be employable in *in silico* mass spectral databases for identification of unknown compounds. Non-limiting system 300 can comprise system 302 and system 318. System 302 can be communicatively, electronically, operatively or otherwise coupled to system 318, and system 302 can comprise software 304. In some embodiments, software 304 can be a command line application based on a Python™ script. In other embodiments, software 304 can be based on other computer programming languages such as C++, JavaScript®, etc. As illustrated in FIGS. 3 - 5, software 304 can comprise matrix computation component 310, mass spectrum component 312, and/or spectral database component 502 illustrated in FIG. 5.

Discussion turns briefly to processor 306, memory 308 and bus 307 of system 302. For example, in one or more embodiments, system 302 can comprise processor 306 (e.g., computer processing unit, microprocessor, classical processor, and/or like processor). In one or more embodiments, a component associated with system 302, as described herein with or without reference to the one or more figures of the one or more embodiments, can comprise one or more computer and/or machine readable, writable and/or executable components and/or instructions that can be executed by processor 306 to enable performance of one or more processes defined by such component(s) and/or instruction(s).

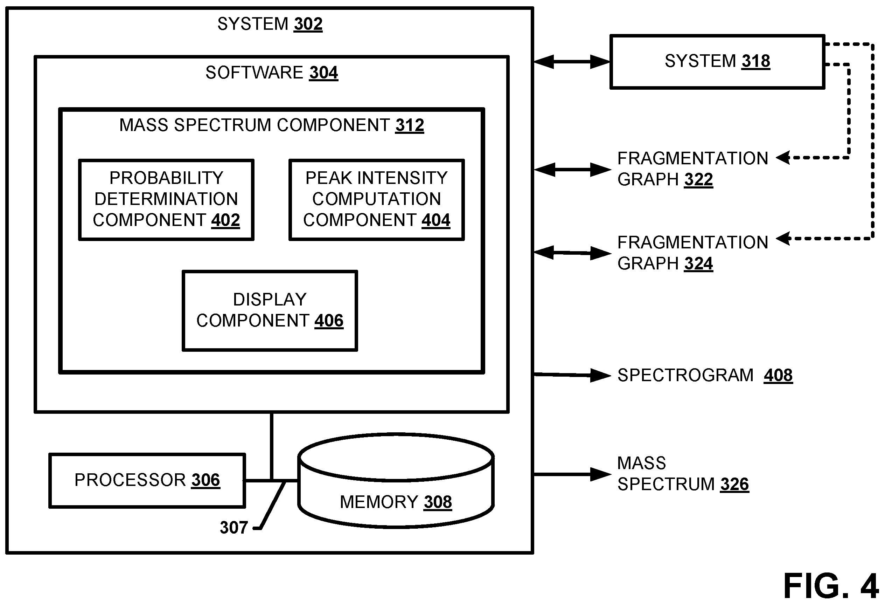

In one or more embodiments, system 302 can comprise a computer-readable memory (e.g., memory 308) that can be operably connected to processor 306. Memory 308 can store computer-executable instructions that, upon execution by processor 306, can cause processor 306 and/or one or more other components of system 302 (e.g., matrix computation component 310, mass spectrum component 312 and/or other components illustrated in FIGS. 4 and 5) to perform one or more actions. In one or more embodiments, memory 308 can store the computer-executable components (e.g., matrix computation component 310, mass spectrum component 312 and/or other components illustrated in FIGS. 4 and 5).

System 302 and/or a component thereof as described herein, can be communicatively, electrically, operatively, optically and/or otherwise coupled to one another via bus 307. Bus 307 can comprise one or more of a memory bus, memory controller, peripheral bus, external bus, local bus, and/or another type of bus that can employ one or more bus architectures. One or more of these examples of bus 307 can be employed. In one or more embodiments, system 302 can be coupled (e.g., communicatively, electrically, operatively, optically and/or like function) to one or more external systems (e.g., a non-illustrated electrical output production system, one or more output targets, an output target controller and/or the like), sources and/or devices (e.g., classical computing devices, communication devices and/or like devices), such as via a network. In one or more embodiments, one or more of the components of system 302 can reside in the cloud, and/or can reside locally in a local computing environment (e.g., at a specified location(s)).

Although not illustrated, system 318 can be a computing system comprising a memory and a processor, similar to system 302.

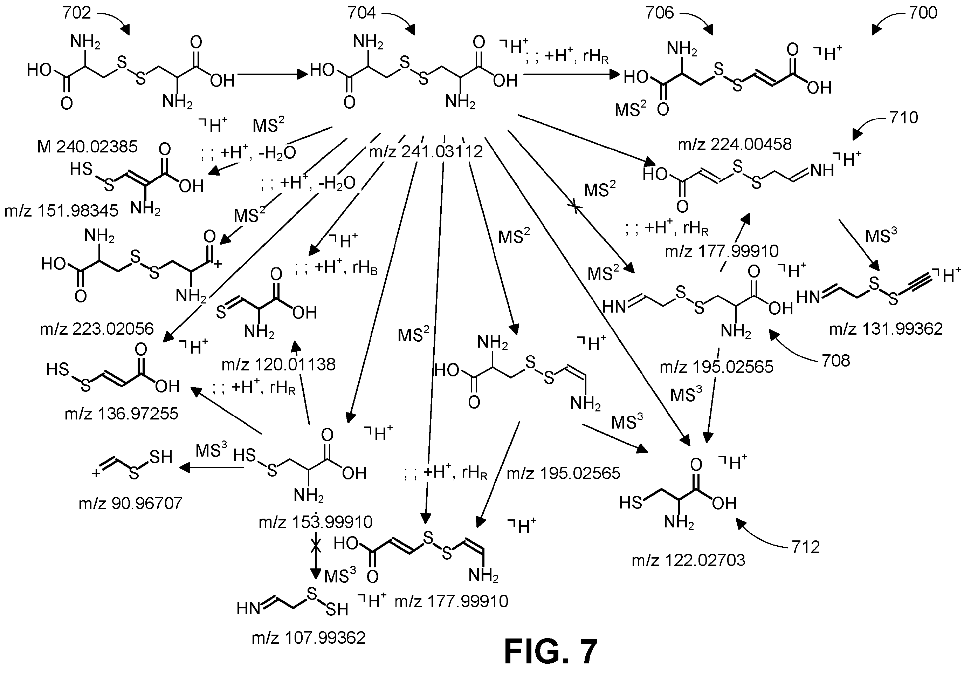

In one or more embodiments, system 302 can generate *in silico* mass spectra for compounds based on fragmentation schemes computed by system 318. For example, system 318 can comprise a software (e.g., Sledge-Hammer or another software) that can simulate the fragmentation (a chemical reaction) of an ionized molecule. For example, an entity (e.g., hardware, software, machine, AI, neural network and/or user) can input information about a root molecule, such as molecule 702 illustrated in FIG. 7, into non-limiting system 300, wherein the software comprised in system 318 can access the information about the root molecule and the software can ionize the root molecule. The information about the root molecule can include a description of the structure of the molecule including the atoms and chemical bonds forming the molecule, and the information can be input into non-limiting system 300 as a MOL-file, International Chemical Identifier (InChi), Simplified Molecular-Input Line-Entry System (SMILES) or another type of data format. In FIG. 7, molecule 704 can represent the *in silico* ionized molecule generated by the software of system 318. After generating the *in silico* ionized molecule, the software of system 318 can simulate a fragmentation of the *in silico* ionized molecule. For example, the software can predict the chemical reactions and ion fragments that can occur based on the fragmentation of the ionized molecule. The chemical reactions and the parent and child ion fragments associated with the fragmentation can be represented in fragmentation graph 322. Fragmentation graph 322 can be written in the JavaScript Object Notation (JSON) format and stored as a file in memory 308 or another memory such as cloud storage on Amazon Web Services® (AWS®) or another cloud storage service.

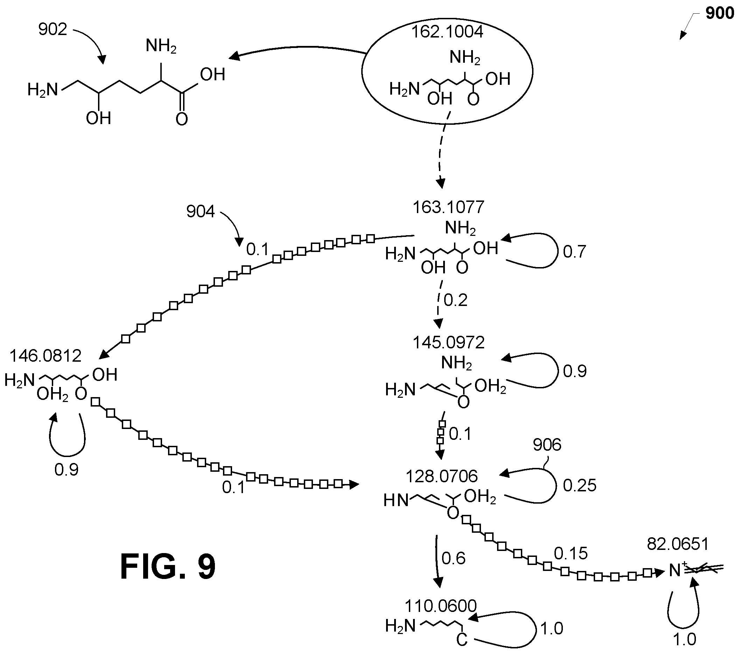

Fragmentation graph 322 can be a directed graph of ion fragments (products) that can result from the fragmentation of the *in silico* ionized molecule, wherein each node can represent an ion fragment and each edge connecting two nodes can represent a chemical reaction that can generate the one or more child ion fragments from a parent ion fragment. Fragmentation graph 322 can be employed by a machine learning software known in the art to compute respective reaction probabilities for the respective chemical reactions described by fragmentation graph 322. For example, the machine learning software can estimate the probability of each chemical reaction occurring and generate fragmentation graph 324, wherein fragmentation graph 324 can comprise the reaction probability values. Thus, fragmentation graph 322 can show a simulation of the chemical reactions and the type of ion fragments that the can be generated via a simulated fragmented of the *in silico* ionized molecule by the software of system 302, and fragmentation graph 324 can identify which chemical reactions from fragmentation graph 322 have a higher probability of occurrence. The machine learning software can add reaction probability values as edge labels and masses of the ion fragments as node labels in fragmentation graph 324. Additionally, the machine learning software can depict the different reaction probabilities as edges having different colors in fragmentation graph 324. For example, FIG. 9 illustrates a portion of a fragmentation graph with reaction probabilities, wherein edges with different reaction probabilities can be visualized by different colors. A pre-processing algorithm that can interact with the machine learning algorithm can add self-going edges to nodes in the fragmentation graph 324. The self-going edges can also be color coded in a different color from the other edges. Self-going edges are artificial edges that indicate that an ion fragment does not further fragment into a child ion fragment. Stated differently, a self-going edge represents a probability that an ion fragment will not break down further or a probability that an ion fragment will generate itself, such that the ion fragment is both the parent ion fragment and the child ion fragment. The self-going edges are added because a chemical reaction that does not occur can still have an associated probability that can be represented by a value.

The machine learning software can be an optimization algorithm based on a neural network. In an embodiment, the machine learning software can be comprised in system 318. In another embodiment, the machine learning software can be comprised in system 302. In yet another embodiment, the machine learning software can be comprised in a system other than system 302 and system 318. Irrespective of the location of the machine learning software, the machine learning software can read the fragmentation JSONs in fragmentation graph 322 via a file-based exchange format from memory 308 or the other memory via localized connections or via the cloud to compute the respective reaction probabilities for the respective chemical reactions. As such, the input to the machine learning software can be the descriptions/digital fingerprints of the chemical reactions from fragmentation graph 322, and the output of the machine learning software can be the respective reaction probabilities/likelihoods of respective chemical reactions. In one or more embodiments, system 318 and/or another system can be part of system 302 rather than being separate computing systems as illustrated in FIG. 3, such that the software that can generate fragmentation graph 322 (e.g., SledgeHammer or another software), the machine learning software that can generate fragmentation graph 324 and software 304 can be comprised within the same system.

Fragmentation graph 324 can also be written in the JSON format and stored as a file in memory 308 or another memory such as cloud storage on AWS® or another cloud storage service. In one or more embodiments, fragmentation graph 324 can be accessed by software 304 to generate *in silico* mass spectra for the root molecule. For example, software 304 can access the respective reaction probabilities in fragmentation graph 324 via a file-based exchange format from memory 308 or the other memory via localized connections or via the cloud. Thereafter, matrix computation component 310 can compute a Markov transition matrix based on the respective reaction probabilities. For example, matrix computation component 310 can represent each reaction probability from fragmentation graph 324 as an element in the Markov transition matrix. In this regard, the Markov transition matrix can be a graph adjacency matrix $A$ comprising value $a_{ij}$ at row $i$ and column $j$, wherein $a_{ij} = 0$, if an ion fragment $i$ does not have $j$ as a child ion fragment (appears as no edge in fragmentation graphs 322 and 324), and wherein $a_{ij} = p_{ij}$, if an ion fragment $i$ produces $j$ as a child ion fragment with probability $p_{ij}$. The Markov transition matrix can be employed by mass spectrum component 312 to generate mass spectrum 326 for the root molecule, as described with reference to FIG. 4, wherein mass spectrum 326 can be an *in silico* mass spectrum.

FIG. 4 illustrates another block diagram of non-limiting system 300, in accordance with various embodiments described herein. Repetitive description of like elements and/or processes employed in respective embodiments is omitted for sake of brevity.

FIG. 4 illustrates additional aspects of non-limiting system 300 with continued reference to FIG. 3.

In one or more embodiments, mass spectrum component 312 can comprise probability determination component 402, peak intensity computation component 404 and display component 406 that can be employed by software 304 to generate mass spectrum 326 for the root molecule. For example, probability determination component 402 can employ the Markov transition matrix to compute a desired state within the fragmentation of the root molecule via a random walk process. For example, probability determination component 402 can express a state of the fragmentation as a state vector having a size equal to the number of ion fragments that can exist at that state, such that multiplying the Markov transition matrix with a state vector representing any state in the fragmentation can generate another state vector that can represent a subsequent state in the fragmentation. Each component or element of a state vector can thus represent a fragment probability for an ion fragment at the corresponding state of the fragmentation. Stated differently, each component or element in the state vector can be a probability at which an ion fragment can occur/probability of the ion fragment being generated at the corresponding state of the fragmentation. For example, the element $S_i$ of the state vector $S$ can be a fragment probability for the ion fragment $i$. Then, one step of state transition from a state $S_t$ to another state $S_{t+1}$ can be expressed by the matrix multiplication given by Equation 1:

$$\text{Equation 1: } S_{t+1} = A \cdot S_t$$

The random walk process can be an iterative process. For example, probability determination component 402 can represent the initial state of the fragmentation as a state vector with a single non-zero element representing the unfragmented ionized molecule. For example, the initial state $S_0$ of the ionized molecule can be described by the exemplary state vector in Equation 2.

$$\text{Equation 2: } S_0 = [1 \ 0 \ ... \ 0]^T$$

Probability determination component 402 can multiply the Markov transition matrix with the state vector from Equation 2 to generate another state vector that can represent a subsequent step in the fragmentation. For example, multiplying the Markov transition matrix with the state vector once (i.e., one step of the random walk process) can generate a new state vector that can represent the second step in the fragmentation, multiplying the Markov transition matrix with the state vectors three times (i.e., three steps of the random walk process) can generate a vector that can represent the fourth step in the fragmentation, and so on. Accordingly, Equation 1 can be more generally written as Equation 3.

$$\text{Equation 3: } S_k = A^k \cdot S_0,$$

wherein $S$ represents a fragmentation state, $S_0$ represents the initial state of the fragmentation wherein the ionized molecule is in the unfragmented state, $A^k$ represents the Markov transition matrix, $k$ represents the number of steps employed in the random walk process, and $S_k$ represents the state of the fragmentation after $k$ steps. $S_0$ can also represent the spectrum of the unfragmented ionized molecule. In this manner, probability determination component 402 can model the fragmentation of the *in silico* ionized molecule as a Markov process based on the random walk process. Each element in the resulting state vector can represent a fragment probability of an ion fragment that can be generated at that state in the fragmentation. Thus, based on the Markov transition matrix, probability determination component 402 can transform the respective reaction probabilities from fragmentation graph 324 into respective fragment probabilities associated with the fragmentation of the ionized molecule.

In one or more embodiments, the fragment probabilities generated by probability determination component 402 can be accessed by peak intensity computation component 404 to generate mass spectrum 326 for the root molecule. For example, peak intensity computation component 404 can compute a sum of fragment probabilities of respective ion fragments resulting from the fragmentation and having identical masses. More specifically, fragmentation graph 324 can comprise several different ion fragments with the same mass, and for each ion fragment resulting from fragmentation of the ionized molecule, peak intensity computation component 404 can compute a sum of respective fragment probabilities of respective ion fragments having identical masses as the ion fragment. Herein, the charge is taken as being equal to 1. The sum of fragment probabilities for an ion fragment represents the peak intensity for the ion fragment, and peak intensities thus computed for ion fragments with unique masses can represent mass spectrum 326. For example, in one or more embodiments, display component 406 can display the peak intensities computed by peak intensity computation component 404 as mass spectrum 326 on a device (e.g., a scientific instrument, a desktop computer, a laptop, a smartphone, etc.). In one or more embodiments, display component 406 can also display the sum of fragment probabilities for each ion fragment having a unique mass as a peak on spectrogram 408. Thus, peak intensity computation component 404 can compute respective fragment probabilities from the respective reaction probabilities in fragmentation graph 324, and further compute mass spectrum 326 for the root molecule from the respective fragment probabilities. Ion fragments with higher fragment probabilities appear as more intense peaks on mass spectrum 326.

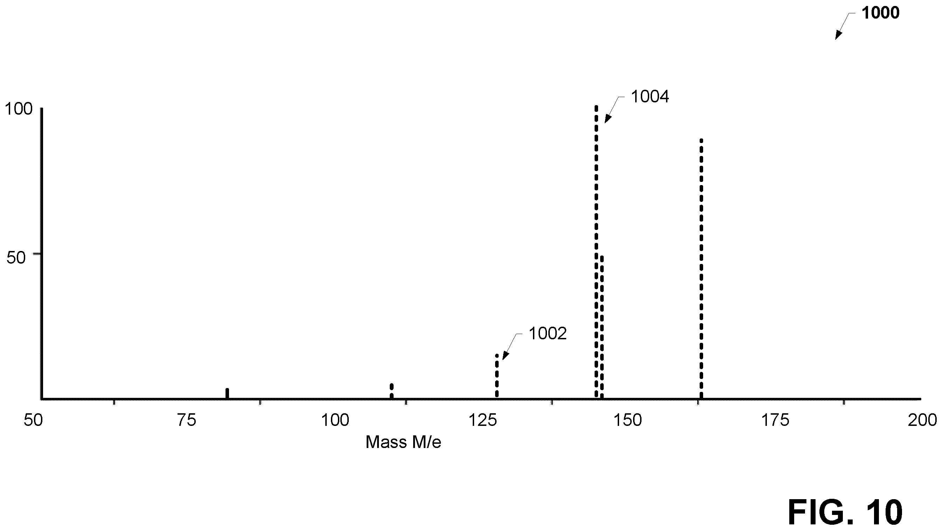

Thus, software 304 can convert reaction probabilities associated with fragmentation of an *in silico* ionized molecule into mass spectrum 326 for a molecule. Software 304 can model the intensities of ion fragments resulting from the fragmentation of the *in silico* ionized molecule. In the mass spectrum, the masses of the ion fragments resulting from fragmentation of the *in silico* ionized molecule can be presented on the X-axis of a two-dimensional (2D) Cartesian coordinate system, as further illustrated in FIG. 10.

FIG. 5 illustrates another block diagram of non-limiting system 300, in accordance with various embodiments described herein. Repetitive description of like elements and/or processes employed in respective embodiments is omitted for sake of brevity.

FIG. 5 illustrates additional aspects of non-limiting system 300 with continued reference to FIGS. 3 and 4.

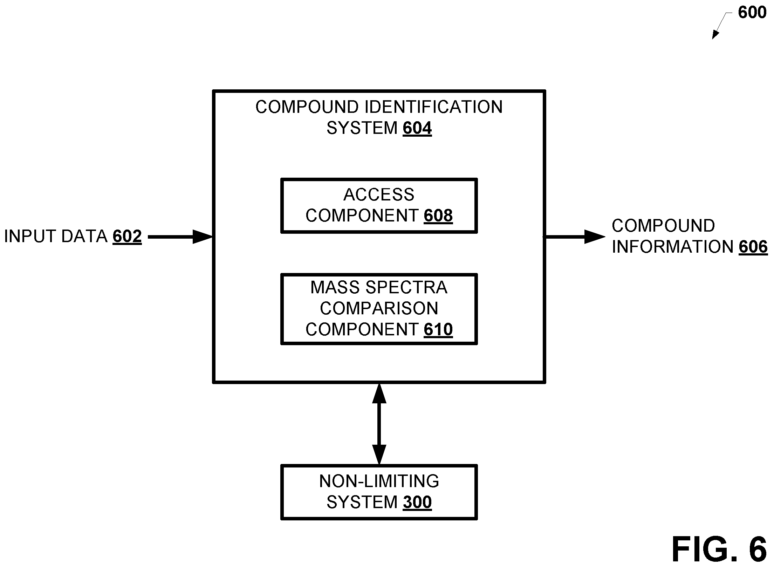

In one or more embodiments, software 304 can further comprise spectral database component 502 that can generate spectral database 504 based on mass spectrum 326, wherein spectral database 504 can be an *in silico* mass spectral database employable for compound identification. For example, spectral database component 502 can compile *in silico* mass spectra generated by mass spectrum component 312 into spectral database 504 and store spectral database 504 in a memory. Thereafter, the spectral database 504 can be accessed by an end entity (e.g., hardware, software, machine, AI, neural network and/or user) as an application on a device (e.g., a scientific instrument, desktop computer, laptop, mobile phones, etc.) or via the cloud environment for compound identification. For example, an end entity can employ spectral database 504 as an *in silico* library of mass spectra generated from compounds, as further explained with reference to FIG. 6.

FIG. 6 illustrates a block diagram of an example, non-limiting system 600 that can employ an *in silico* mass spectral database to identify unknown compounds, in accordance with various embodiments described herein. Repetitive description of like elements and/or processes employed in respective embodiments is omitted for sake of brevity.

With continued reference to non-limiting system 300, compound identification system 604 can be employed by an end entity (e.g., hardware, software, machine, AI, neural network and/or user) as a library of *in silico* mass spectra generated from compounds (e.g., chemical compounds such as small molecules, etc.) to identify unknown compounds. For example, the end entity can input information about the unknown compound as input data 602 into compound identification system 604 via a graphical user interface (GUI). Compound identification system 604 can be communicatively, electrically, operatively or otherwise coupled to non-limiting system 300, and compound identification system 604 can generate compound information 606, wherein compound information 606 can comprise information about the unknown compound.

More specifically, an unknown compound can refer to a compound suspected to be a lipid. For example, the unknown compound can be measured by a chemist, scientist, laboratory personnel or another entity, via a mass spectrometer, to generate an experimental mass spectrum for the unknown compound. The experimental mass spectrum can indicate that the unknown compound comprises a set of several lipids (e.g., hundreds or thousands). To confirm that the unknown compound is a lipid, data from the experimental mass spectrum can be input as input data 602 into compound identification system 604. Access component 608 can access input data 602, and mass spectra comparison component 610 can check spectral database 504 for relevant *in silico* mass spectra. If the *in silico* mass spectra for the lipids exist in spectral database 504, mass spectra comparison component 610 can check spectral database 504 to directly identify the unknown compound. If the *in silico* mass spectra for the lipids are absent from spectral database 504, mass spectra comparison component 610 can employ non-limiting system 300 to first the generate the *in silico* mass spectra for the lipids based on a description of the unknown compound. Thereafter, mass spectra comparison component 610 can compare the experimental mass spectrum for the unknown compound with the *in silico* mass spectra to identify the unknown compound.

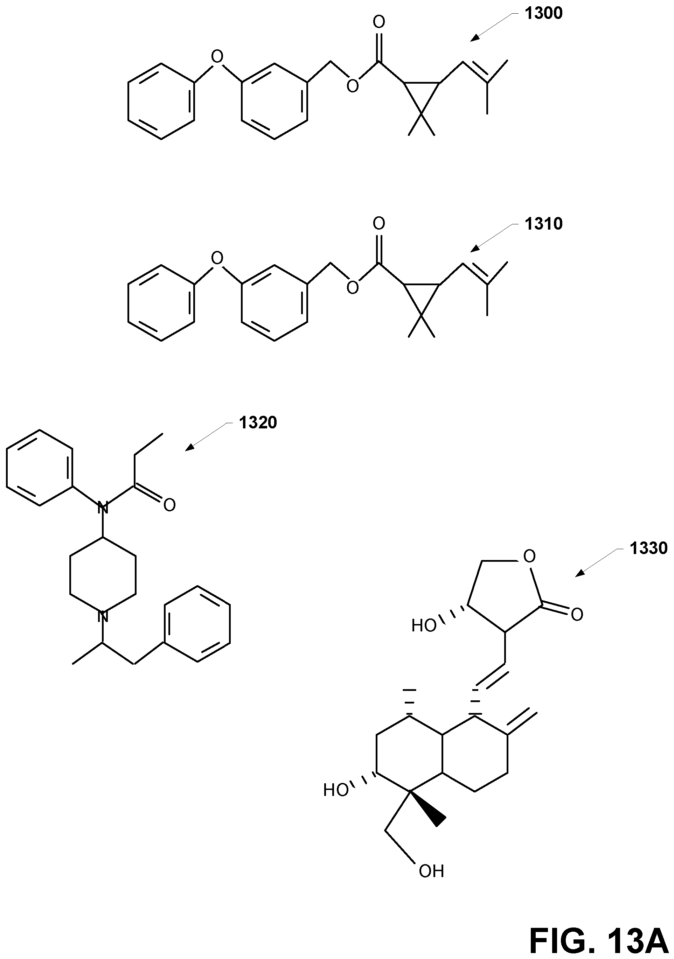

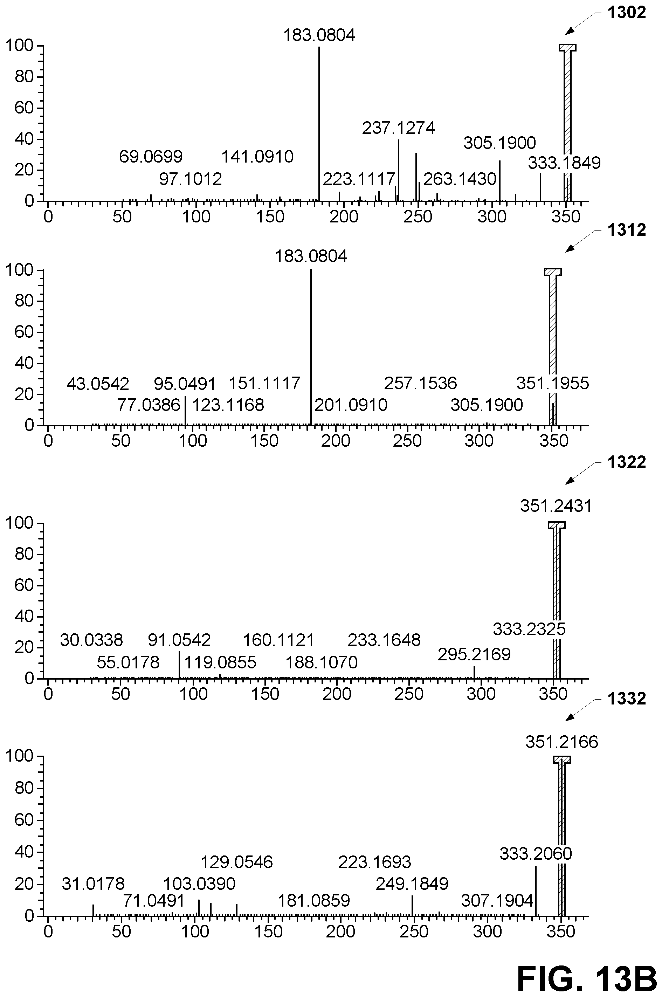

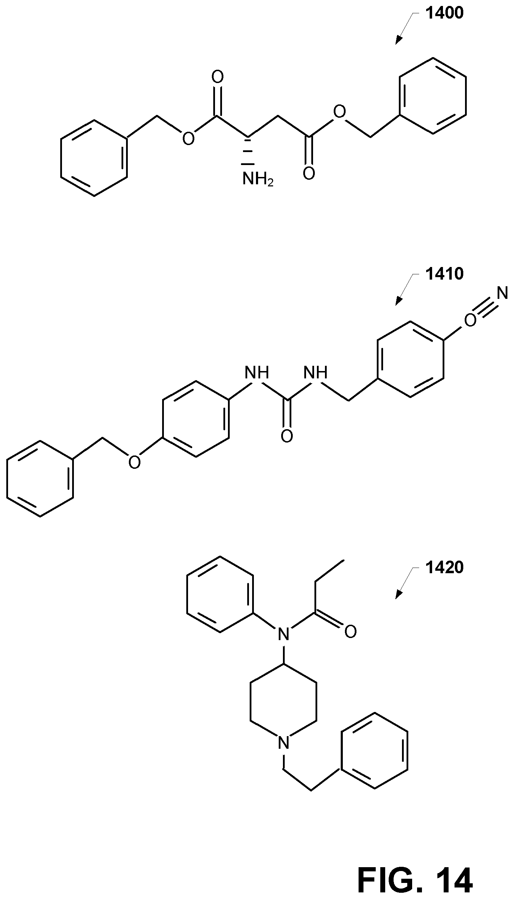

Mass spectra comparison component 610 can generate the results of the comparison as compound information 606. Mass spectra comparison component 610 can employ different measurements such as cosine score, Median Denver similarity score, etc. to compare the experimental and mass spectra, as further explained with reference to FIGS. 13 and 14. Thus, non-limiting system 300 can be employed in multiple capacities. In one or more embodiments, non-limiting system 300 and non-limiting system 600 can be employed in scientific instruments to generate *in silico* mass spectra and identify compounds based on the *in silico* mass spectra. For example, a first scientific instrument can employ non-limiting system 300 to generate *in silico* mass spectra of compounds, a second scientific instrument can employ non-limiting system 600 to generate *in silico* mass spectra and perform compound identification of compounds, and so on.

FIG. 7 illustrates a diagram of an example, non-limiting fragmentation graph 700 that can be generated via a simulated fragmentation of an ionized molecule, in accordance with various embodiments described herein. Repetitive description of like elements and/or processes employed in respective embodiments is omitted for sake of brevity.

With continued reference to at least FIGS. 3 - 6, non-limiting fragmentation graph 700 illustrates an example of fragmentation graph 322 generated by non-limiting system 300. For example, information about molecule 702 can be processed by a software (e.g., SledgeHammer or another software) of system 318 to generate molecule 704, wherein molecule 704 can be an *in silico* ionized molecule. Thereafter, the software of system 318 can simulate a fragmentation of molecule 704 to generate the ion fragments illustrated in non-limiting fragmentation graph 700. During the fragmentation, molecule 704 can fragment into one or more child ion fragments, and each of the one or more ion fragments can further fragment into one or more child ion fragments. For example, molecules 706, 708, 710 and 712 can be respective child ion fragments of molecule 704, and molecules 710 and 712 can further be respective child ion fragments of molecule 708. Each ion fragment can be a node in non-limiting fragmentation graph 700, and each edge connecting two nodes can be the corresponding chemical reaction. Further, non-limiting fragmentation graph 700 can depict each ion fragment by its molecular structure and indicate the mass (m), charge (z) and/or mass-to-charge ratios of the complete ion. It should be noted that for ease of illustration, non-limiting fragmentation graph 700 illustrates a simplified subgraph of the original fragmentation graph that can be much larger in reality and comprise tens of thousands of nodes.

The number of fragmentation steps simulated by the software of system 318 can be limited for various reasons. Firstly, simulating an infinite number of fragmentation steps can consume a significant amount of time (several years in some cases), be slower, and consume vast amounts of memory. Secondly, the number of fragmentation steps simulate the energy imparted to the system of the unfragmented ionized molecule, and adding more fragmentation steps (more energy) can break down many of the child ion fragments into smaller fragments, keeping only the smallest fragments that cannot be broken down further. As a result, only the parent ion fragments that cannot generate any more child ion fragments can be visualized by the *in silico* mass spectrum with the fragment probabilities of the parent ion fragments being zero.

Thus, to address one or more potential disadvantages resultant from not limiting the number of fragmentation steps, in some embodiments, the number of fragmentation steps simulated by the software of system 318 can be limited based on a target simulated energy. For example, in some embodiments, the number of fragmentation steps simulated by the software of system 318 can be limited to a value that results in a target simulated energy that is less than or equal to a defined threshold.

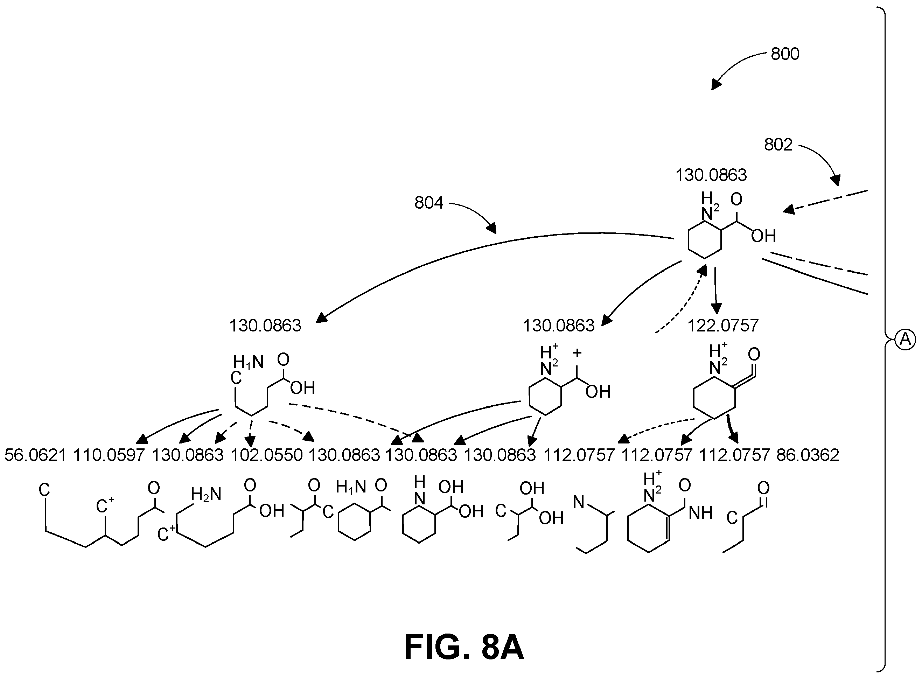

FIGS. 8A - 8C illustrate a diagram of an example, non-limiting fragmentation graph 800 with reaction probabilities that can be generated via a machine learning software, in accordance with various embodiments described herein. Repetitive description of like elements and/or processes employed in respective embodiments is omitted for sake of brevity.

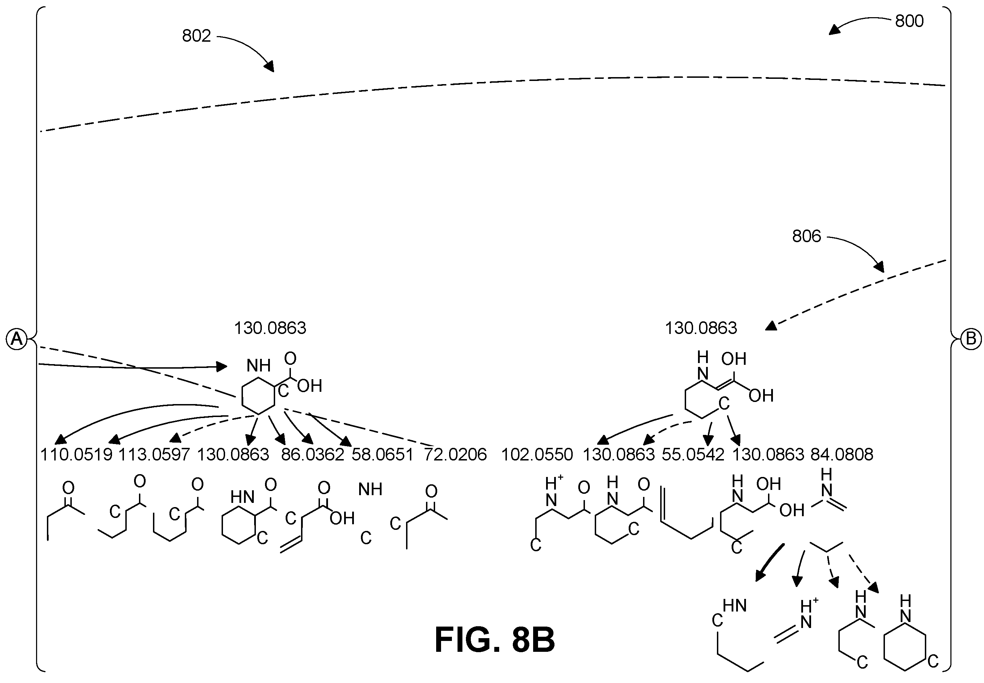

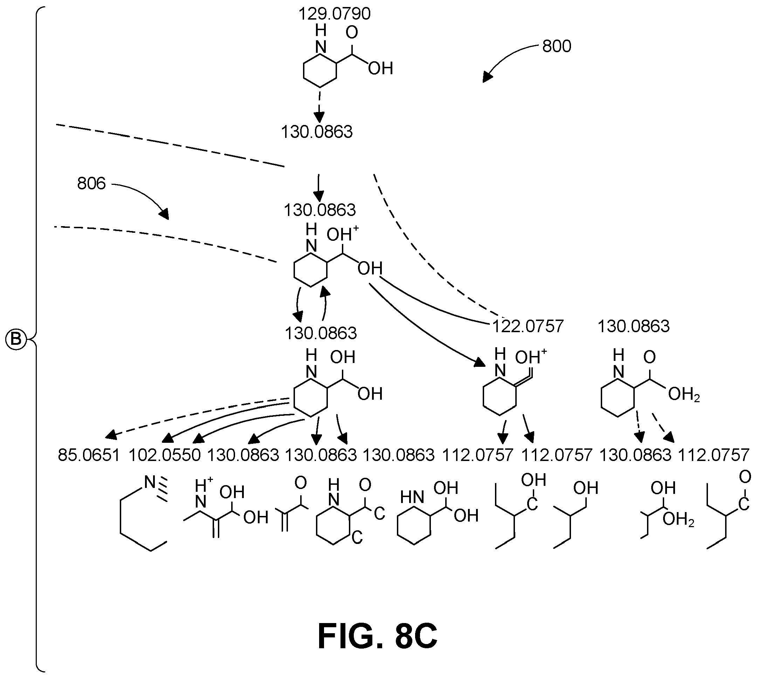

FIGS. 8A- 8C collectively illustrate different subsections of non-limiting fragmentation graph 800, with a first subsection illustrated in FIG. 8A, a second subsection illustrated in FIG. 8B and a third subsection illustrated in FIG. 8C. With continued reference to at least FIGS. 3 - 6, non-limiting fragmentation graph 800 illustrates an example of fragmentation graph 324 generated by non-limiting system 300. For example, fragmentation graph 322 can be accessed by a machine learning software comprised in system 318, system 302 or another system, and the machine learning software can compute reaction probabilities for the chemical reactions in fragmentation graph 322. As a result, a new fragmentation graph such as non-limiting fragmentation graph 800 can be generated wherein the reaction probabilities can be visualized as edges with different colors. In other words, the machine learning software can apply different colors to the edges in fragmentation graph 322 according to the reaction probabilities represented by the edges. For example, edges representing chemical reactions with a low probability (e.g., 0.0 reaction probability) can be depicted in green color whereas edges representing chemical reactions with a high probability (e.g., 1.0 reaction probability) can be depicted in red color. Edges 802, 804 and 806 are examples of such edges. As previously described, a pre-processing algorithm that can interact with the machine learning software can also add self-going edges to the new fragmentation graph, and machine learning software can compute the reaction probability value for each edge. This is further illustrated in FIG. 9. It should be appreciated that in FIGS. 8A- 8C and 9, edges corresponding to different colors are depicted with different patterns, wherein like patterns correspond to like colors.

FIG. 9 illustrates a diagram of an example, non-limiting fragmentation graph 800 with reaction probabilities and self-going edges that can be generated via a machine learning software, in accordance with various embodiments described herein. Repetitive description of like elements and/or processes employed in respective embodiments is omitted for sake of brevity.

With continued reference to FIGS. 3 - 6, non-limiting fragmentation graph 900 illustrates another example of fragmentation graph 324 with reaction probabilities and self-going edges depicted as edges corresponding to different colors. All self-going edges can be represented by a single color (e.g., grey color or another colors) that can be different from the colors applied to the other edges (e.g., red, green, etc.). Additionally, non-limiting fragmentation graph 900 also illustrates the respective reaction probability values for the respective edges. For example, edge 904 can represent a chemical reaction with a reaction probability of 0.1, whereas self-going edge 906 can represent a chemical reaction that does not occur and indicate that the probability of the corresponding ion fragment generating itself as a child ion fragment is 0.25. Recall that a self-going edge represents a case where no further fragmentation occurs. The sum of outgoing reaction probabilities for an ion fragment should be equal to 1. That is, the sum of reaction probabilities of each chemical reaction that can occur from a parent ion fragment should be equal to 1. The reaction probability for each chemical reaction can be computed by the machine learning software based on the fact that the sum of reaction probabilities for all outgoing edges representing chemical reactions should be equal to 1. Thereafter, the machine learning software can normalize the sum of reaction probabilities to be equal to 1. It should be noted that for ease of illustration, both non-limiting fragmentation graph 800 of FIGS. 8A - 8C, and non-limiting fragmentation graph 900 illustrate simplified subgraphs of the original fragmentation graphs that can be much larger in reality than illustrated. Additionally, in FIGS. 8A - 8C and 9, edges having different colors are depicted as edges with different patterns, wherein like patterns represent like colors.

Non-limiting fragmentation graph 900 illustrates an exemplary fragmentation scheme of the compound 5-Hydroxylysine. For example, information about 5-Hydroxylysine can be input into non-limiting system 300 to first generate an *in silico* representation of an ionized molecule for 5-Hydroxylysine, followed by simulating a fragmentation of the ionized molecule. At this stage, 5-Hydroxylysine can be an unknown compound. The molecular structure of 5-Hydroxylysine is illustrated at 902 as a magnified view. In one or more embodiments, system 302 can employ mass spectrum component 312 to generate an *in silico* mass spectrum (e.g., mass spectrum 326) based on non-limiting fragmentation graph 900. For example, matrix computation component 310 of software 304 can access non-limiting fragmentation graph 900, and matrix computation component 310 can compute a Markov transition matrix based on the reaction probabilities in non-limiting fragmentation graph 900. In this regard, the Markov transition matrix is another representation of non-limiting fragmentation graph 900. For example, matrix computation component 310 can compute the Markov transition matrix according to Equation 4 based on non-limiting fragmentation graph 900. In the Markov transition matrix, the element values along the diagonal extending from the top left element to the bottom right element (i.e., 0.7 to 1) represent the reaction probabilities associated with the self-going edges.

$$\text{Equation 4: } A = \begin{bmatrix} 0.7 & 0 & 0 & 0 & 0 & 0 \\ 0.1 & 0.9 & 0 & 0 & 0 & 0 \\ 0.2 & 0 & 0.9 & 0 & 0 & 0 \\ 0 & 0.1 & 0.1 & 0.25 & 0 & 0 \\ 0 & 0 & 0 & 0.6 & 1 & 0 \\ 0 & 0 & 0 & 0.15 & 0 & 1 \end{bmatrix}$$

The Markov transition matrix can be employed by probability determination component 402 in a random walk process to generate an *in silico* mass spectrum for 5-Hydroxylysine by transforming the respective reaction probabilities from non-limiting fragmentation graph 900 into respective fragment probabilities. For example, probability determination component 402 can compute the state vector according to Equation 5 to represent the initial state of fragmentation of 5-Hydroxylysine.

$$\text{Equation 5: } S_0 = \begin{bmatrix} 1 \\ 0 \\ 0 \\ 0 \\ 0 \\ 0 \end{bmatrix}$$

Probability determination component 402 can multiply the Markov transition matrix with the state vector to generate a subsequent state in the fragmentation according to Equation 3. Herein, the state vector resulting from three steps of random walk is presented by Equation 6. In one or more embodiments, the number of steps of the random walk process executed by probability determination component 402 of software 304 can be predefined by an entity (e.g., hardware, software, machine, AI, neural network and/or user). For example, in some implementations, nine to eleven steps of random walk can be executed, whereas in other implementations, additional or fewer steps of random walk can be executed. By employing the Markov transition matrix in the random walk process, the fragmentation of 5-Hydroxylysine can be modeled as a Markov process and non-limiting fragmentation graph 900 can be modeled as a Markov chain.

$$\text{Equation 6:} \begin{bmatrix} 0.7 & 0 & 0 & 0 & 0 & 0 \\ 0.1 & 0.9 & 0 & 0 & 0 & 0 \\ 0.2 & 0 & 0.9 & 0 & 0 & 0 \\ 0 & 0.1 & 0.1 & 0.25 & 0 & 0 \\ 0 & 0 & 0 & 0.6 & 1 & 0 \\ 0 & 0 & 0 & 0.15 & 0 & 1 \end{bmatrix}^3 \cdot \begin{bmatrix} 1 \\ 0 \\ 0 \\ 0 \\ 0 \\ 0 \end{bmatrix} = \begin{bmatrix} 0.343 \\ 0.193 \\ 0.386 \\ 0.056 \\ 0.018 \\ 0.005 \end{bmatrix}$$

Each element in the resulting matrix can represent the respective fragment probabilities corresponding to the respective fragment ions generated during the fragmentation of 5-Hydroxylysine. In one or more embodiments, peak intensity computation component 404 can access the fragment probabilities and compute a sum of fragment probabilities for ion fragments having identical masses. Peak intensity computation component 404 can compute a sum of fragment probabilities for each ion fragment having a unique mass. For example, if ion fragments A, B, C and D have identical masses, peak intensity computation component 404 can compute a first sum of respective fragment probabilities of ion fragments A, B, C and D, if ion fragments E, F and G have identical masses that are different from those of ion fragments A, B, C and D, peak intensity computation component 404 can compute a second sum of respective fragment probabilities of ion fragments E, F and G, and so on. The sum for fragment probabilities corresponding to an ion fragment can represent the intensity of the ion fragment. The intensities thus computed for ion fragments with unique masses can be displayed by display component 406 as the *in silico* mass spectrum at a device (e.g., a scientific instrument, a desktop computer, a laptop, a smartphone, etc.). The sum of fragment probabilities can also appear as a peak on a spectrogram. The *in silico* mass spectrum for 5-Hydroxylysine is illustrated in FIG. 10.

FIG. 10 illustrates a diagram of an example, non-limiting mass spectrum 1000, in accordance with various embodiments described herein. Repetitive description of like elements and/or processes employed in respective embodiments is omitted for sake of brevity.

Non-limiting mass spectrum 1000 can be an *in silico* mass spectrum generated by mass spectrum component 312 for a molecule of 5-Hydroxylysine. Non-limiting mass spectrum 1000 can be displayed at a device (e.g., a scientific instrument, a desktop computer, a laptop, a smartphone, etc.) by display component 406, and peaks 1002, 1004 and other such peaks can represent the peak intensities of ion fragments with unique masses. In non-limiting mass spectrum 1000, the X-axis can indicate the mass-to-charge ratio (m/e or m/z) for an ion fragment and the Y-axis can indicate the relative abundance or intensity of ion fragments with a particular mass-to-charge ratio.

In some embodiments, only an *in silico* mass spectrum such as non-limiting mass spectrum 1000 can be displayed by display component 406 at a device of an end entity (e.g., hardware, software, machine, AI, neural network and/or user). In other embodiments, a spectrogram such as spectrogram 408 of FIGS. 4 and 5 and/or fragmentation graphs such as non-limiting fragmentation graph 700 of FIG. 7, non-limiting fragmentation graph 800 of FIGS. 8A- 8C and non-limiting fragmentation graph 900 of FIG. 9 can also be displayed by display component 406 at a device of the end entity. Such fragmentation graphs can be a secondary output by non-limiting system 300 of FIGS. 3 - 6. The end entity can view the different fragmentation graphs and the *in silico* mass spectra to analyze how a fragmentation was simulated, the different chemical reactions and ion fragments that were generated along the process, and so on. In one or more embodiments, the *in silico* mass spectra can be displayed to the end entity as a final output. If an *in silico* mass spectrum is displayed as it is being generated, then at the zeroth step, only a single peak can be visible in the *in silico* mass spectrum and as more steps of the Markov process are executed, the intensities of parent ion fragments can get smaller while the intensities of the child ion fragments can rise. In one or more embodiments, the end entity can interact, via a GUI, with the various outputs (e.g., spectrogram 408, non-limiting fragmentation graph 700, non-limiting fragmentation graph 800, non-limiting fragmentation graph 900 of FIG. 9) that can be displayed by display component 406 to collect, store or analyze the data comprised in such outputs.

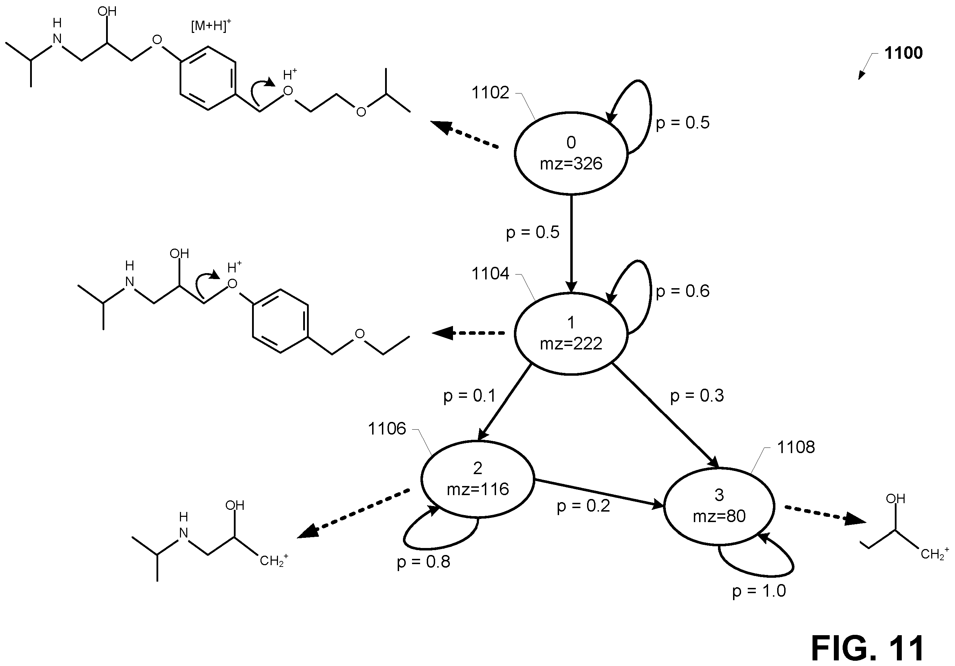

FIG. 11 illustrates a diagram of an example, non-limiting fragmentation graph 1100 showing a random walk process, in accordance with various embodiments described herein. Repetitive description of like elements and/or processes employed in respective embodiments is omitted for sake of brevity.

With continued reference to FIGS. 3 - 6, FIG. 11 is intended to illustrate the random walk process executed by probability determination component 402. Specifically, FIG. 11 illustrates how a defined number of steps in the random walk process can be executed by probability determination component 402 of FIG. 4, in the context of a fragmentation graph, by employing a Markov transition matrix representative of the fragmentation graph. In this regard, FIG. 11 also illustrates how the fragmentation of an ionized molecule modeled as the Markov process can occur in a mass spectrometry instrument.

Non-limiting fragmentation graph 1100 can be a fragmentation graph with reaction probabilities, such as non-limiting fragmentation graph 800 of FIGS. 8A- 8C or non-limiting fragmentation graph 900. The random walk process executed by probability determination component 402 can begin at node 1102 with an unfragmented molecule, and after three steps of random walk following the reaction probability corresponding to each edge (similar to throwing a dice and randomly generating a number) an ion fragment with a mass-to-charge ratio (m/z) of about 222, with the mass in the m/z being measured in Daltons, can be generated at node 1104. This can correspond to a point for the specific mass on a mass spectrum. Other random walks beginning at node 1104 and following the reaction probability at each edge can generate an ion fragment with an m/z value of about 80 at node 1108 and an ion fragment with an m/z value of about 116 at node 1106. Yet another random walk can lead to the unfragmented molecule with an m/z value of about 326 at node 1102. Each random walk can correspond to a point on a mass spectrum with the m/z value of the ion fragment thus generated. As such, repeating the random walk process (e.g., 10,000 times) can generate a histogram such as non-limiting histogram 1200 that plots the masses of each ion fragment generated during the fragmentation.

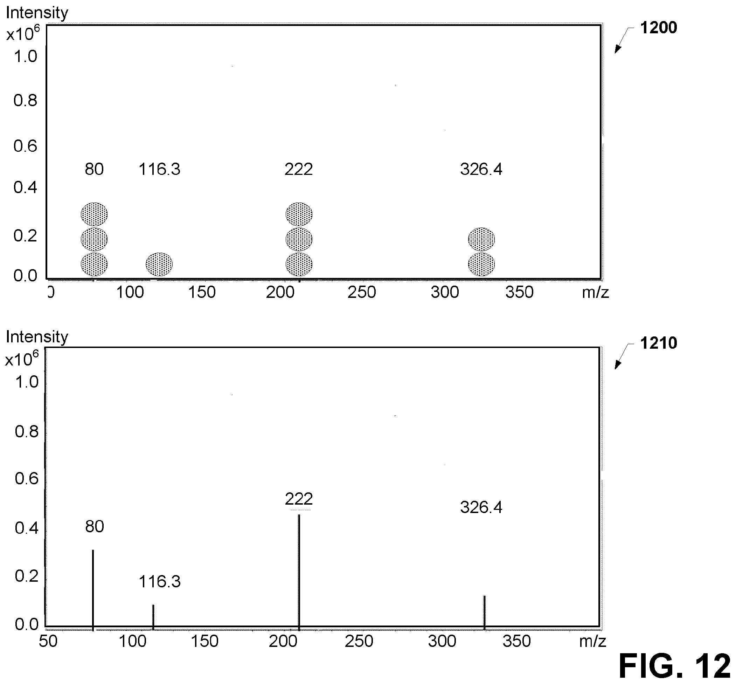

FIG. 12 illustrates an example, non-limiting histogram 1200 and an example, non-limiting mass spectrum 1210 based on a random walk process involving a Markov transition matrix, in accordance with various embodiments described herein. Repetitive description of like elements and/or processes employed in respective embodiments is omitted for sake of brevity.

Non-limiting histogram 1200 illustrates a sample distribution and histogram based on the random walk process detailed with reference to FIG. 11, and non-limiting mass spectrum 1210 illustrates the corresponding mass spectrum as a stationary distribution. Recall that the sum of fragment probabilities of ion fragments having identical masses represent the intensity for those ion fragments. For example, the fragment probabilities of two ion fragments with the same mass can be added together by peak intensity computation component 404 to represent the intensities of each of the two ion fragments. Thus, in non-limiting histogram 1200, three points for the m/z value of 80 can indicate that presence of ion fragments with mass 80 is more intense in the fragmentation visualized by non-limiting fragmentation graph 1100.

In one or more embodiments, a histogram such as non-limiting histogram 1200 can be generated by display component 406 in addition to non-limiting mass spectrum 1210. In one or more embodiments, non-limiting histogram 1200 can also be an output displayed at a device accessible to an end entity (e.g., hardware, software, machine, AI, neural network and/or user).

As discussed in one or more embodiments herein, employing a Markov transition matrix can be a computationally less intensive technique of constructing an *in silico* mass spectrum, as compared to conventional approaches. For example, the respective fragment probabilities for respective ion fragments can also be computed by finding all paths to each specific ion fragment in a fragmentation graph such as non-limiting fragmentation graph 1100. Then, the fragment probability for an ion fragment can be generated by computing the product (multiplication) of all probabilities along the path, followed by a summation through all paths leading to the node representing the ion fragment. However, the number of paths leading to a node can be large, and the number can grow exponentially as the number of paths increase. Storing all such paths in memory can be computationally demanding. On the contrary, the random walk process employed in the various embodiments herein avoids the use of paths altogether. Further, although fragmentation graphs can be large, representing the fragmentation graphs as sparse vectors/matrices can also reduce the memory consumption in the process. For example, a typical large fragmentation graph can have about 90,000 nodes and 300,000 edges. Thus, the ratio of non-zero entries in a corresponding Markov transition matrix (adjacency matrix) is $\frac{300{,}000}{(90{,}000*90{,}000)}$, which equates to only about a 0.0037% fill rate. Finally, the matrix-based approach disclosed herein is supported by graphics processing units (GPUs), which can accelerate the computing speed involved in generating an *in silico* mass spectrum. Many existing computation libraries such as, for example, PyTorch®, support operations on sparse matrices, even with GPUs. In this regard, the technique disclosed herein can be more versatile as compared to conventional techniques that are not supported by GPUs.

FIG. 13A illustrates example, non-limiting molecules 1300, 1310, 1320 and 1330 and FIG. 13B illustrates example, non-limiting mass spectra 1302, 1312, 1322 and 1332 corresponding to the molecules illustrated in FIG. 13A, in accordance with various embodiments described herein. Repetitive description of like elements and/or processes employed in respective embodiments is omitted for sake of brevity.

As discussed elsewhere herein, non-limiting system 300, and specifically system 302 of FIGS. 3 - 6, can be employed to generate *in silico* mass spectra for compounds such as small molecules, wherein the *in silico* mass spectra can be employed in *in silico* mass spectral databases and synthetic libraries such as spectral database 504 to identify compounds. In this regard, FIG. 12 illustrates an example of compound identification via non-limiting system 300.

For example, molecule 1300 can be an unknown molecule and mass spectrum 1302 can be an experimental mass spectrum generated by measuring molecule 1300 via a mass spectrometer. Molecule 1310 represents a Phenothrin molecule, and mass spectrum 1312 is the *in silico* mass spectrum generated by system 302 for molecule 1310. Molecule 1320 represents an α-Methylfentanyl molecule, and mass spectrum 1322 is the *in silico* mass spectrum generated by system 302 for molecule 1320. Molecule 1330 represents an Andrographolide molecule, and mass spectrum 1332 is the *in silico* mass spectrum generated by system 302 for molecule 1330. Mass spectra 1312, 1322 and 1332 can be stored in a memory (e.g., memory 308) as part of spectral database 504. In one or more embodiments, compound identification system 604 can be employed to identify molecule 1300 based on mass spectrum 1302. For example, in one or more embodiments, information about mass spectrum 1302 can be input to compound identification system 604 by an entity (e.g., hardware, software, machine, AI, neural network and/or user) and compound identification system 604 can compare mass spectrum 1302 to the *in silico* mass spectra in spectral database 504 to identify molecule 1300 or confirm suspicions about what molecule 1300 could be. An output thus generated by compound identification system 604 can indicate that molecule 1310 is a close or exact match to molecule 1300, whereas molecules 1320 and 1330 are not similar to molecule 1300. This can indicate that molecule 1300 is Phenothrin.

In one or more embodiments, compound identification system 604 can employ different measurements such as cosine score, Median Denver similarity score, etc. to compare the experimental and mass spectra. The Median Denver similarity score is similar to the cosine score wherein the peaks in the mass spectra are weighted with the mass (m) and charge (z) values and similarities in peaks and peak intensities are compared.

FIGS. 14 - 16 illustrate example, non-limiting test data and test results based on the matrix-based approach employed to generate *in silico* mass spectra, in accordance with various embodiments described herein. Repetitive description of like elements and/or processes employed in respective embodiments is omitted for sake of brevity.

The Median Denver similarity score was also employed to test the performance of software 304 of FIGS. 3 - 6. The resulting Median Denver similarity score between the experimental and predicted (*in silico*) spectra was 0.8 for the spectra from the Normalized Collision Energy (NCE) range 5 - 35 and 0.7 for the spectra from the NCE range 40 - 80. In mass spectrometry, the NCE range indicates the amount of energy applied to ions to induce fragmentation of the ions.

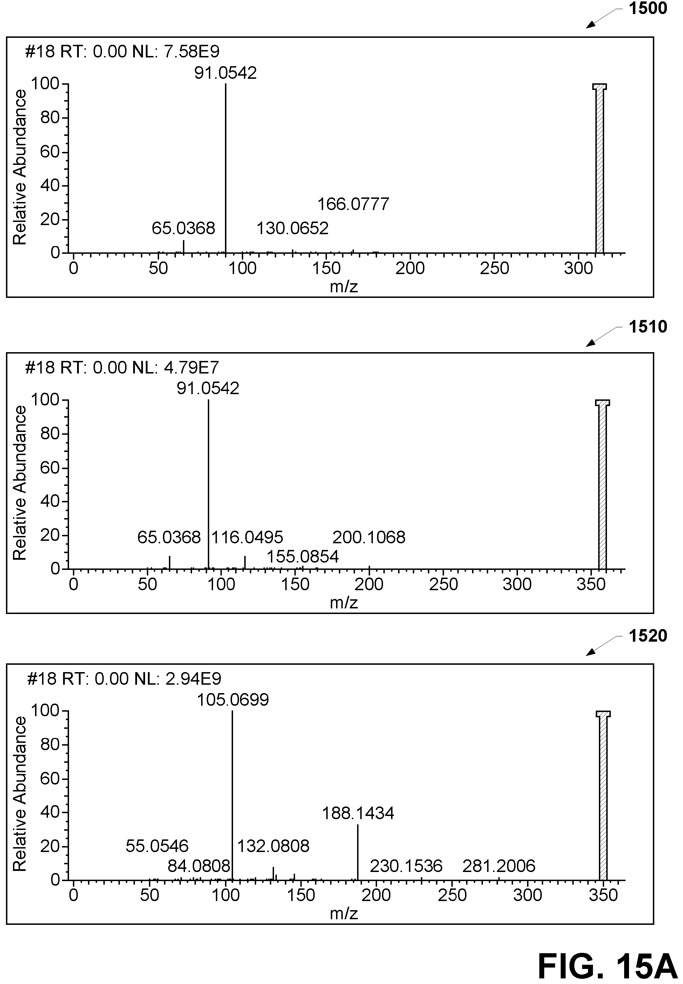

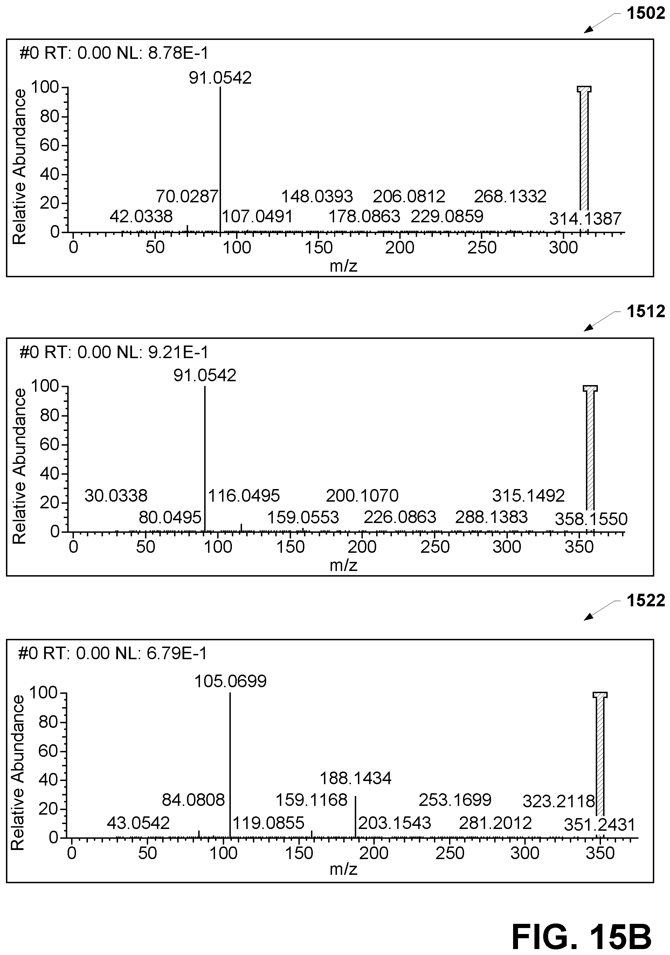

For example, in FIG. 14, molecule 1400 represents a (D)-Dibenzyl 2-aminosuccinate molecule, molecule 1410 represents an N-[4-(Benzyloxy)phenyl]-N'-(4-cyanobenzyl)urea molecule, and molecule 1420 represents a Butyryl fentanyl molecule. Mass spectra 1500, 1510 and 1520 of FIG. 15A respectively represent experimental mass spectra for molecules 1400, 1410 and 1420, and mass spectra 1502, 1512 and 1522 of FIG. 15B respectively represent *in silico* mass spectra for molecules 1400, 1410 and 1420. Mass spectra 1500, 1510 and 1520 were generated by a mass spectrometer, whereas mass spectra 1502, 1512 and 1522 were generated by software 304. To test the performance of software 304, mass spectrum 1500 was compared with mass spectrum 1502, mass spectrum 1510 was compared with mass spectrum 1512, and mass spectrum 1520 was compared with mass spectrum 1522. The Median Denver similarity scores thus obtained are presented in Table 1.

Table 1:

| Mass spectra compared | Score |
|---|---|
| Mass spectra 1500 and 1502 | 0.74 |
| Mass spectra 1510 and 1512 | 0.81 |
| Mass spectra 1500 and 1502 | 0.82 |

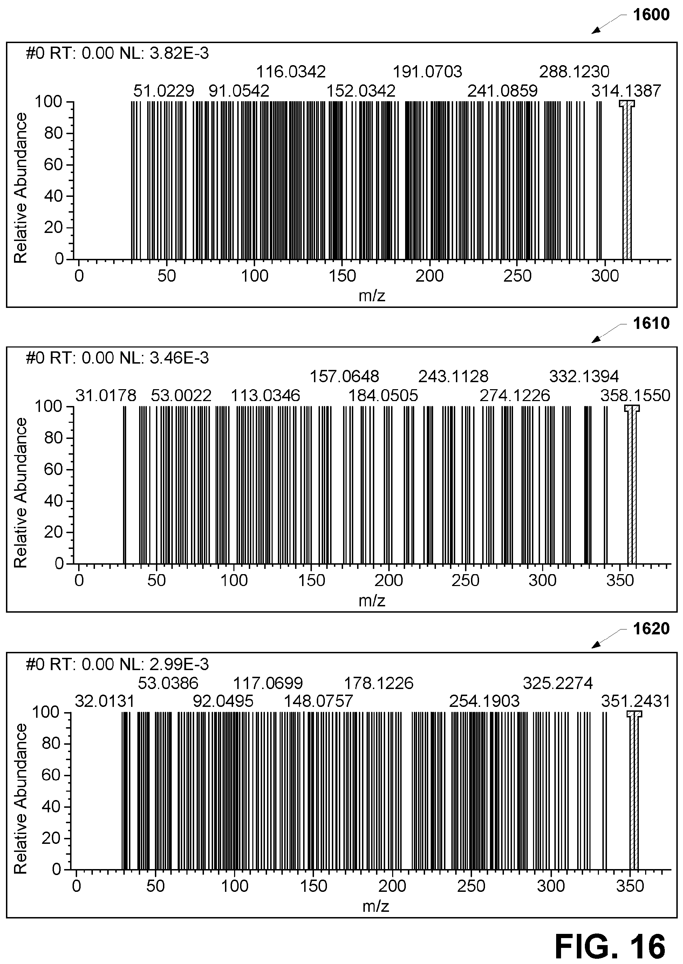

FIG. 16 illustrates example, non-limiting barcode graphs 1600, 1610 and 1620, in accordance with various embodiments described herein. Non-limiting barcode graphs 1600, 1610 and 1620 respectively correspond to molecules 1400, 1410 and 1420. In mass spectrometry, barcode graphs are employed to represent the presence and relative abundance of ion fragments and can be employed to identify compounds present in samples.

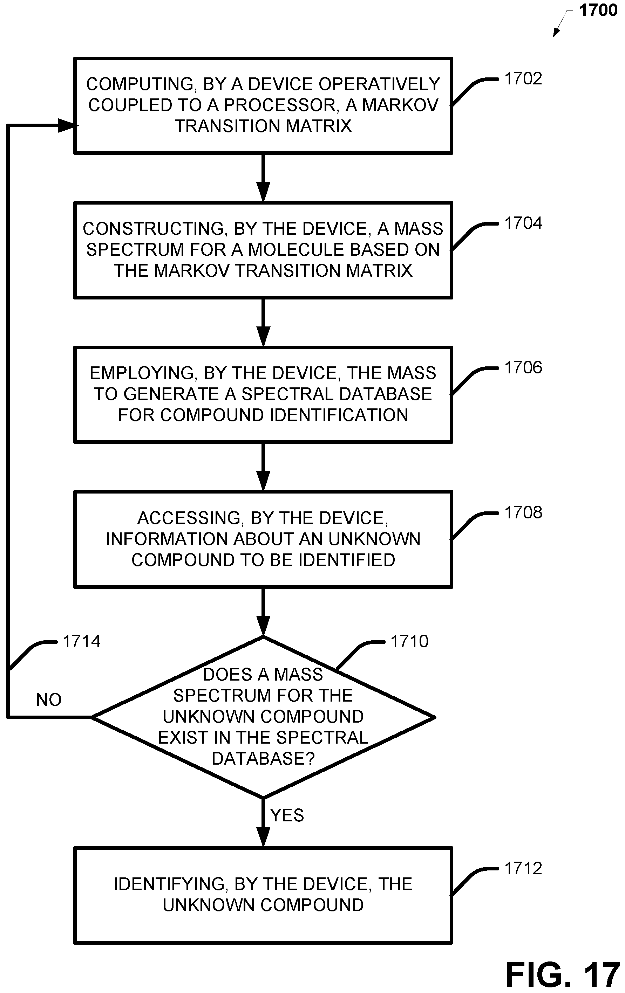

FIG. 17 illustrates a flow diagram of an example, non-limiting method 1700 that can construct *in silico* mass spectra for compounds, in accordance with various embodiments described herein. Repetitive description of like elements and/or processes employed in respective embodiments is omitted for sake of brevity.

At 1702, the non-limiting method 1700 can comprise computing (e.g., by matrix computation component 310), by a device operatively coupled to a processor, a Markov transition matrix.

At 1704, the non-limiting method 1700 can comprise constructing (e.g., by mass spectrum component 312), by the device, a mass spectrum for a molecule based on the Markov transition matrix.

In one or more embodiments, the mass spectrum can be employed to generate a spectral database for compound identification. For example:

At 1706, the non-limiting method 1700 can comprise employing (e.g., by spectral database component 502), by

EP 4 708 303 A1

the device, the mass spectrum to generate a spectral database.

At 1708, the non-limiting method 1700 can comprise accessing (e.g., by access component 608), by the device, information about an unknown compound to be identified.

At 1710, the non-limiting method 1700 can comprise determining (e.g., by mass spectra comparison component 610), by the device, whether a mass spectrum for the unknown compound exists in the spectral database.

If yes, then at 1712, the non-limiting method 1700 can comprise identifying (e.g., by mass spectra comparison component 610), by the device, the unknown compound by comparing the information about the unknown compound with mass spectra comprised in the spectral database.

If not, then at 1714, the non-limiting method 1700 can return to 1702.

For simplicity of explanation, the computer-implemented and non-computer-implemented methodologies provided herein are depicted and/or described as a series of acts. It is to be understood that the subject innovation is not limited by the acts illustrated and/or by the order of acts, for example acts can occur in one or more orders and/or concurrently, and with other acts not presented and described herein. Furthermore, not all illustrated acts can be utilized to implement the computer-implemented and non-computer-implemented methodologies in accordance with the described subject matter. Additionally, the computer-implemented methodologies described hereinafter and throughout this specification are capable of being stored on an article of manufacture to enable transporting and transferring the computer-implemented methodologies to computers. The term article of manufacture, as used herein, is intended to encompass a computer program accessible from any computer-readable device or storage media.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

FIG. 18 depicts an example GUI 1800 that can be used in the performance of some or all of the support methods or techniques disclosed herein, in accordance with various embodiments. As noted above, the GUI 1800 can be provided on a display device (e.g., the display device 1910 discussed herein with reference to FIG. 19) of a computing device (e.g., the computing device 1900 discussed herein with reference to FIG. 19) of a scientific instrument support system (e.g., the scientific instrument support system 2000 discussed herein with reference to FIG. 20), and a user (e.g., chemist, scientist, laboratory personnel or another user) can interact with the GUI 1800 using any suitable input device (e.g., any of the input devices included in the other I/O devices 1912 discussed herein with reference to FIG. 19) and input technique (e.g., movement of a cursor, motion capture, facial recognition, gesture detection, voice recognition, actuation of buttons, etc.).

The GUI 1800 can include a data display region 1802, a data analysis region 1804, a scientific instrument control region 1806, and a settings region 1808. The particular number and arrangement of regions depicted in FIG.18 is simply illustrative, and any number and arrangement of regions, including any desired features, can be included in a GUI 1800.

The data display region 1802 can display data generated by a scientific instrument (e.g., the scientific instrument 2010 discussed herein with reference to FIG. 20). For example, the data display region 1802 can display data representations such as those of FIGS. 7 - 10, 13, 15 and 16.

The data analysis region 1804 can display the results of data analysis (e.g., the results of analyzing the data illustrated in the data display region 1802 and/or other data). For example, the data analysis region 1804 can display the results of analyzing the data illustrated in FIGS. 10, 13, 15 and 16. In some embodiments, the data display region 1802 and the data analysis region 1804 can be combined in the GUI 1800 (e.g., to include data output from a scientific instrument, and some analysis of the data, in a common graph or region).

The scientific instrument control region 1806 can include options that allow the user (e.g., chemist, scientist, laboratory personnel or another user) to control a scientific instrument (e.g., the scientific instrument 2010 discussed herein with reference to FIG. 20). For example, the scientific instrument control region 1806 can include configurable parameters that govern the operations of such scientific instruments.

The settings region 1808 can include options that allow the user to control the features and functions of the GUI 1800 (and/or other GUIs) and/or perform common computing operations with respect to the data display region 1802 and data analysis region 1804 (e.g., saving data on a storage device, such as the storage device 1904 discussed herein with reference to FIG. 19, sending data to another user, labeling data, etc.). For example, the settings region 1808 can include options that can allow the user to perform computing operations with respect to the data representation and results displayed by data display region 1802 and/or data analysis region 1804.

As noted above, the scientific instrument support module 102 can be implemented by one or more computing devices. FIG. 19 is a block diagram of a computing device 1900 that can perform some or all of the scientific instrument support methods disclosed herein, in accordance with various embodiments. In some embodiments, the scientific instrument support module 102 can be implemented by a single computing device 1900 or by multiple computing devices 1900. Further, as discussed below, a computing device 1900 (or multiple computing devices 1900) that implements the scientific instrument support module 102 can be part of one or more of the scientific instrument 2010, the user local computing device 2020, the service local computing device 2030, or the remote computing device 2040 of FIG. 20.

The computing device 1900 of FIG. 19 is illustrated as having a number of components, but any one or more of these components may be omitted or duplicated, as suitable for the application and setting. In some embodiments, some or all of the components included in the computing device 1900 can be attached to one or more motherboards and enclosed in a housing (e.g., including plastic, metal, and/or other materials). In some embodiments, some these components can be fabricated onto a single system-on-a-chip (SoC) (e.g., an SoC may include one or more processing devices 1902 and one or more storage devices 1904). Additionally, in various embodiments, the computing device 1900 may not include one or more of the components illustrated in FIG. 19, but may include interface circuitry (not shown) for coupling to the one or more components using any suitable interface (e.g., a Universal Serial Bus (USB) interface, a High-Definition Multimedia Interface (HDMI) interface, a Controller Area Network (CAN) interface, a Serial Peripheral Interface (SPI) interface, an Ethernet interface, a wireless interface, or any other appropriate interface). For example, the computing device 1900 may not include a display device 1910, but may include display device interface circuitry (e.g., a connector and driver circuitry) to which a display device 1910 may be coupled.

The computing device 1900 can include a processing device 1902 (e.g., one or more processing devices). As used herein, the term "processing device" can refer to any device or portion of a device that processes electronic data from registers and/or memory to transform that electronic data into other electronic data that may be stored in registers and/or memory. The processing device 1902 can include one or more digital signal processors (DSPs), application-specific integrated circuits (ASICs), central processing units (CPUs), graphics processing units (GPUs), cryptoprocessors (specialized processors that execute cryptographic algorithms within hardware), server processors, or any other suitable processing devices.

The computing device 1900 can include a storage device 1904 (e.g., one or more storage devices). The storage device 1904 can include one or more memory devices such as random access memory (RAM) (e.g., static RAM (SRAM) devices, magnetic RAM (MRAM) devices, dynamic RAM (DRAM) devices, resistive RAM (RRAM) devices, or conductive-bridging RAM (CBRAM) devices), hard drive-based memory devices, solid-state memory devices, networked drives, cloud drives, or any combination of memory devices. In some embodiments, the storage device 1904 can include memory that shares a die with a processing device 1902. In such an embodiment, the memory may be used as cache memory and may include embedded dynamic random access memory (eDRAM) or spin transfer torque magnetic random access memory (STT-MRAM), for example. In some embodiments, the storage device 1904 can include non-transitory computer readable media having instructions thereon that, when executed by one or more processing devices (e.g., the processing device 1902), cause the computing device 1900 to perform any appropriate ones of or portions of the methods disclosed herein.

The computing device 1900 can include an interface device 1906 (e.g., one or more interface devices 1906). The interface device 1906 can include one or more communication chips, connectors, and/or other hardware and software to govern communications between the computing device 1900 and other computing devices. For example, the interface device 1906 can include circuitry for managing wireless communications for the transfer of data to and from the computing device 1900. The term "wireless" and its derivatives may be used to describe circuits, devices, systems, methods, techniques, communications channels, etc., that may communicate data through the use of modulated electromagnetic radiation through a nonsolid medium. The term does not imply that the associated devices do not contain any wires, although in some embodiments they might not. Circuitry included in the interface device 1906 for managing wireless communications may implement any of a number of wireless standards or protocols, including but not limited to Institute for Electrical and Electronic Engineers (IEEE) standards including Wi-Fi (IEEE 802.11 family), IEEE 802.16 standards (e.g., IEEE 802.16-2005 Amendment), Long-Term Evolution (LTE) project along with any amendments, updates, and/or revisions (e.g., advanced LTE project, ultra mobile broadband (UMB) project (also referred to as "3GPP2"), etc.). In some embodiments, circuitry included in the interface device 1906 for managing wireless communications may operate in accordance with a Global System for Mobile Communication (GSM), General Packet Radio Service (GPRS), Universal Mobile Telecommunications System (UMTS), High Speed Packet Access (HSPA), Evolved HSPA (E-HSPA), or LTE network. In some embodiments, circuitry included in the interface device 1906 for managing wireless communications may operate in accordance with Enhanced Data for GSM Evolution (EDGE), GSM EDGE Radio Access Network (GERAN), Universal Terrestrial Radio Access Network (UTRAN), or Evolved UTRAN (E-UTRAN). In some embodiments, circuitry included in the interface device 1906 for managing wireless communications may operate in accordance with Code Division Multiple Access (CDMA), Time Division Multiple Access (TDMA), Digital Enhanced Cordless Telecommunications (DECT), Evolution-Data Optimized (EV-DO), and derivatives thereof, as well as any other wireless protocols that are designated as 3G, 4G, 5G, and beyond. In some embodiments, the interface device 1906 can include one or more antennas (e.g., one or more antenna arrays) to receipt and/or transmission of wireless communications.

In some embodiments, the interface device 1906 can include circuitry for managing wired communications, such as electrical, optical, or any other suitable communication protocols. For example, the interface device 1906 can include circuitry to support communications in accordance with Ethernet technologies. In some embodiments, the interface device 1906 can support both wireless and wired communication, and/or may support multiple wired communication protocols and/or multiple wireless communication protocols. For example, a first set of circuitry of the interface device 1906 can be dedicated to shorter-range wireless communications such as Wi-Fi or Bluetooth, and a second set of circuitry of the interface device 1906 can be dedicated to longer-range wireless communications such as global positioning system (GPS), EDGE, GPRS, CDMA, WiMAX, LTE, EV-DO, or others. In some embodiments, a first set of circuitry of the interface device 1906 can be dedicated to wireless communications, and a second set of circuitry of the interface device 1906 can be dedicated to wired communications.

The computing device 1900 can include battery/power circuitry 1908. The battery/power circuitry 1908 can include one or more energy storage devices (e.g., batteries or capacitors) and/or circuitry for coupling components of the computing device 1900 to an energy source separate from the computing device 1900 (e.g., alternating current line power).

The computing device 1900 can include a display device 1910 (e.g., multiple display devices). The display device 1910 can include any visual indicators, such as a heads-up display, a computer monitor, a projector, a touchscreen display, a liquid crystal display (LCD), a light-emitting diode display, or a flat panel display.

The computing device 1900 can include other input/output (I/O) devices 1912. The other I/O devices 1912 can include one or more audio output devices (e.g., speakers, headsets, earbuds, alarms, etc.), one or more audio input devices (e.g., microphones or microphone arrays), location devices (e.g., GPS devices in communication with a satellite-based system to receive a location of the computing device 1900, as known in the art), audio codecs, video codecs, printers, sensors (e.g., thermocouples or other temperature sensors, humidity sensors, pressure sensors, vibration sensors, accelerometers, gyroscopes, etc.), image capture devices such as cameras, keyboards, cursor control devices such as a mouse, a stylus, a trackball, or a touchpad, bar code readers, Quick Response (QR) code readers, or radio frequency identification (RFID) readers, for example.

The computing device 1900 can have any suitable form factor for its application and setting, such as a handheld or mobile computing device (e.g., a cell phone, a smart phone, a mobile internet device, a tablet computer, a laptop computer, a netbook computer, an ultrabook computer, a personal digital assistant (PDA), an ultra mobile personal computer, etc.), a desktop computing device, or a server computing device or other networked computing component.

One or more computing devices implementing any of the scientific instrument support modules or methods disclosed herein may be part of a scientific instrument support system. FIG. 20 is a block diagram of an example scientific instrument support system 2000 in which some or all of the scientific instrument support methods disclosed herein may be performed, in accordance with various embodiments. The scientific instrument support modules, methods or techniques disclosed herein (e.g., the scientific instrument support module 102 of FIG. 1, computer-implemented method 200 of FIG. 2, non-limiting system 300 of FIGS. 3 - 5, non-limiting system 600 of FIG. 6, non-limiting method 1700 of FIG. 17) can be implemented by one or more of the scientific instrument 2010, the user local computing device 2020, the service local computing device 2030, or the remote computing device 2040 of the scientific instrument support system 2000.

Any of the scientific instrument 2010, the user local computing device 2020, the service local computing device 2030, or the remote computing device 2040 may include any of the embodiments of the computing device 1900 discussed herein with reference to FIG. 19, and any of the scientific instrument 2010, the user local computing device 2020, the service local computing device 2030, or the remote computing device 2040 can take the form of any appropriate ones of the embodiments of the computing device 1900 discussed herein with reference to FIG. 19.

The scientific instrument 2010, the user local computing device 2020, the service local computing device 2030, or the remote computing device 2040 can each include a processing device 2002, a storage device 2004, and an interface device 2006. The processing device 2002 can take any suitable form, including the form of any of the processing devices 1902 discussed herein with reference to FIG. 19, and the processing devices 2002 included in different ones of the scientific instrument 2010, the user local computing device 2020, the service local computing device 2030, or the remote computing device 2040 may take the same form or different forms. The storage device 2004 may take any suitable form, including the form of any of the storage devices 1904 discussed herein with reference to FIG. 19, and the storage devices 2004 included in different ones of the scientific instrument 2010, the user local computing device 2020, the service local computing device 2030, or the remote computing device 2040 can take the same form or different forms. The interface device 2006 may take any suitable form, including the form of any of the interface devices 1906 discussed herein with reference to FIG. 19, and the interface devices 2006 included in different ones of the scientific instrument 2010, the user local computing device 2020, the service local computing device 2030, or the remote computing device 2040 may take the same form or different forms.

The scientific instrument 2010, the user local computing device 2020, the service local computing device 2030, and the remote computing device 2040 may be in communication with other elements of the scientific instrument support system 2000 via communication pathways 2008. The communication pathways 2008 may communicatively couple the interface devices 2006 of different ones of the elements of the scientific instrument support system 2000, as shown, and may be wired or wireless communication pathways (e.g., in accordance with any of the communication techniques discussed herein with reference to the interface devices 1906 of the computing device 1900 of FIG. 19). The particular scientific instrument support system 2000 depicted in FIG. 20 includes communication pathways between each pair of the scientific instrument 2010, the user local computing device 2020, the service local computing device 2030, and the remote computing device 2040, but this "fully connected" implementation is simply illustrative, and in various embodiments, various ones of the communication pathways 2008 may be absent. For example, in some embodiments, a service local computing device 2030 may not have a direct communication pathway 2008 between its interface device 2006 and the interface device 2006 of the scientific instrument 2010, but may instead communicate with the scientific instrument 2010 via the communication pathway 2008 between the service local computing device 2030 and the user local computing device 2020 and the communication pathway 2008 between the user local computing device 2020 and the scientific instrument 2010.

The scientific instrument 2010 may include any appropriate scientific instrument, such as a scientific instrument employing non-limiting system 300 or non-limiting system 600.

The user local computing device 2020 can be a computing device (e.g., in accordance with any of the embodiments of the computing device 2000 discussed herein) that is local to a user of the scientific instrument 2010. In some embodiments, the user local computing device 2020 can also be local to the scientific instrument 2010, but this need not be the case; for example, a user local computing device 2020 that is in a user's home or office may be remote from, but in communication with, the scientific instrument 2010 so that the user may use the user local computing device 2020 to control and/or access data from the scientific instrument 2010. In some embodiments, the user local computing device 2020 may be a laptop, smartphone, or tablet device. In some embodiments the user local computing device 2020 can be a portable computing device.

The service local computing device 2030 may be a computing device (e.g., in accordance with any of the embodiments of the computing device 1900 discussed herein) that is local to an entity that services the scientific instrument 2010. For example, the service local computing device 2030 can be local to a manufacturer of the scientific instrument 2010 or to a third-party service company. In some embodiments, the service local computing device 2030 can communicate with the scientific instrument 2010, the user local computing device 2020, and/or the remote computing device 2040 (e.g., via a direct communication pathway 2008 or via multiple "indirect" communication pathways 2008, as discussed above) to receive data regarding the operation of the scientific instrument 2010, the user local computing device 2020, and/or the remote computing device 2040 (e.g., the results of self-tests of the scientific instrument 2010, calibration coefficients used by the scientific instrument 2010, the measurements of sensors associated with the scientific instrument 2010, etc.). In some embodiments, the service local computing device 2030 can communicate with the scientific instrument 2010, the user local computing device 2020, and/or the remote computing device 2040 (e.g., via a direct communication pathway 2008 or via multiple "indirect" communication pathways 2008, as discussed above) to transmit data to the scientific instrument 2010, the user local computing device 2020, and/or the remote computing device 2040 (e.g., to update programmed instructions, such as firmware, in the scientific instrument 2010, to initiate the performance of test or calibration sequences in the scientific instrument 2010, to update programmed instructions, such as software, in the user local computing device 2020 or the remote computing device 2040, etc.). A user of the scientific instrument 2010 may utilize the scientific instrument 2010 or the user local computing device 2020 to communicate with the service local computing device 2030 to report a problem with the scientific instrument 2010 or the user local computing device 2020, to request a visit from a technician to improve the operation of the scientific instrument 2010, to order consumables or replacement parts associated with the scientific instrument 2010, or for other purposes.

The remote computing device 2040 may be a computing device (e.g., in accordance with any of the embodiments of the computing device 1900 discussed herein) that is remote from the scientific instrument 2010 and/or from the user local computing device 2020. In some embodiments, the remote computing device 2040 may be included in a datacenter or other large-scale server environment. In some embodiments, the remote computing device 2040 may include network-attached storage (e.g., as part of the storage device 2004). The remote computing device 2040 may store data generated by the scientific instrument 2010, perform analyses of the data generated by the scientific instrument 2010 (e.g., in accordance with programmed instructions), facilitate communication between the user local computing device 2020 and the scientific instrument 2010, and/or facilitate communication between the service local computing device 2030 and the scientific instrument 2010.

In some embodiments, one or more of the elements of the scientific instrument support system 2000 illustrated in FIG. 20 may not be present. Further, in some embodiments, multiple ones of various ones of the elements of the scientific instrument support system 2000 of FIG. 20 can be present. For example, a scientific instrument support system 2000 can include multiple user local computing devices 2020 (e.g., different user local computing devices 2020 associated with different users or in different locations). In another example, a scientific instrument support system 2000 may include multiple scientific instruments 2010, all in communication with service local computing device 2030 and/or a remote computing device 2040; in such an embodiment, the service local computing device 2030 may monitor these multiple scientific instruments 2010, and the service local computing device 2030 may cause updates or other information may be "broadcast" to multiple scientific instruments 2010 at the same time. Different ones of the scientific instruments 2010 in a scientific instrument support system 2000 may be located close to one another (e.g., in the same room) or farther from one another (e.g., on different floors of a building, in different buildings, in different cities, etc.). In some embodiments, a scientific instrument 2010 may be connected to an Internet-of-Things (IoT) stack that allows for command and control of the scientific instrument 2010 through a web-based application, a virtual or augmented reality application, a mobile application, and/or a desktop application. Any of these applications may be accessed by a user operating the user local computing device 2020 in communication with the scientific instrument 2010 by the intervening remote computing device 2040. In some embodiments, a scientific instrument 2010 may be sold by the manufacturer along with one or more associated user local computing devices 2020 as part of a local scientific instrument computing unit 2012.

In some embodiments, different ones of the scientific instruments 2010 included in a scientific instrument support system 2000 may be different types of scientific instruments 2010; for example, one scientific instrument 2010 can be a mass spectrum generation device, while another scientific instrument 2010 can be a compound identification instrument. In some such embodiments, the remote computing device 2040 and/or the user local computing device 2020 can combine data from different types of scientific instruments 2010 included in a scientific instrument support system 2000.

In various instances, machine learning algorithms or models can be implemented in any suitable way to facilitate any suitable aspects described herein. To facilitate some of the above-described machine learning aspects of various embodiments, consider the following discussion of artificial intelligence (AI). Various embodiments described herein can employ artificial intelligence to facilitate automating one or more features or functionalities. The components can employ various AI-based schemes for carrying out various embodiments/examples disclosed herein. In order to provide for or aid in the numerous determinations (e.g., determine, ascertain, infer, calculate, predict, prognose, estimate, derive, forecast, detect, compute) described herein, components described herein can examine the entirety or a subset of the data to which it is granted access and can provide for reasoning about or determine states of the system or environment from a set of observations as captured via events or data. Determinations can be employed to identify a specific context or action, or can generate a probability distribution over states, for example. The determinations can be probabilistic; that is, the computation of a probability distribution over states of interest based on a consideration of data and events. Determinations can also refer to techniques employed for composing higher-level events from a set of events or data.

Such determinations can result in the construction of new events or actions from a set of observed events or stored event data, whether or not the events are correlated in close temporal proximity, and whether the events and data come from one or several event and data sources. Components disclosed herein can employ various classification (explicitly trained (e.g., via training data) as well as implicitly trained (e.g., via observing behavior, preferences, historical information, receiving extrinsic information, and so on)) schemes or systems (e.g., support vector machines, neural networks, expert systems, Bayesian belief networks, fuzzy logic, data fusion engines, and so on) in connection with performing automatic or determined action in connection with the claimed subject matter. Thus, classification schemes or systems can be used to automatically learn and perform a number of functions, actions, or determinations.

A classifier can map an input attribute vector, $z = (z_1, z_2, z_3, z_4, z_n)$, to a confidence that the input belongs to a class, as by $f(z) = confidence(class).$ Such classification can employ a probabilistic or statistical-based analysis (e.g., factoring into the analysis utilities and costs) to determinate an action to be automatically performed. A support vector machine (SVM) can be an example of a classifier that can be employed. The SVM operates by finding a hyper-surface in the space of possible inputs, where the hyper-surface attempts to split the triggering criteria from the nontriggering events. Intuitively, this makes the classification correct for testing data that is near, but not identical to training data. Other directed and undirected model classification approaches include, e.g., naïve Bayes, Bayesian networks, decision trees, neural networks, fuzzy logic models, or probabilistic classification models providing different patterns of independence, any of which can be employed. Classification as used herein also is inclusive of statistical regression that is utilized to develop models of priority.

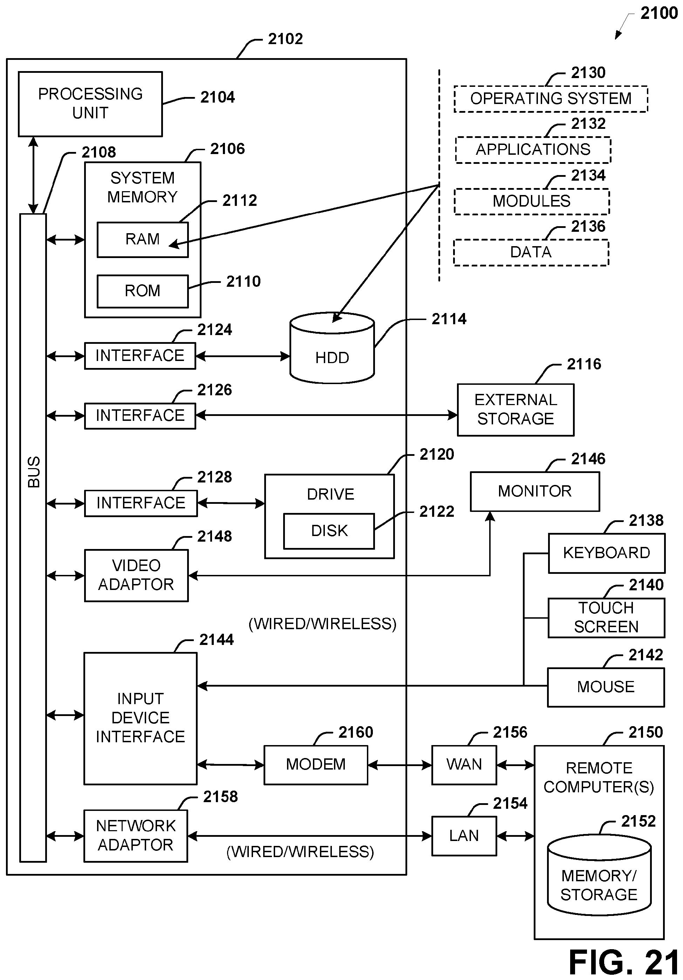

In order to provide additional context for various embodiments described herein, FIG. 21 and the following discussion are intended to provide a brief, general description of a suitable computing environment 2100 in which the various embodiments described herein can be implemented. While the embodiments have been described above in the general context of computer-executable instructions that can run on one or more computers, those skilled in the art will recognize that the embodiments can be also implemented in combination with other program modules or as a combination of hardware and software.

Generally, program modules include routines, programs, components, data structures, etc., that perform particular tasks or implement particular abstract data types. Moreover, those skilled in the art will appreciate that the inventive methods can be practiced with other computer system configurations, including single-processor or multi-processor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can be also practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data or unstructured data.

Computer-readable storage media can include, but are not limited to, random access memory (RAM), read only memory (ROM), electrically erasable programmable read only memory (EEPROM), flash memory or other memory technology, compact disk read only memory (CD-ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible or non-transitory media which can be used to store desired information. In this regard, the terms "tangible" or "non-transitory" herein as applied to storage, memory or computer-readable media, are to be understood to exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

With reference again to FIG. 21, the example environment 2100 for implementing various embodiments of the aspects described herein includes a computer 2102, the computer 2102 including a processing unit 2104, a system memory 2106 and a system bus 2108. The system bus 2108 couples system components including, but not limited to, the system memory 2106 to the processing unit 2104. The processing unit 2104 can be any of various commercially available processors. Dual microprocessors and other multi-processor architectures can also be employed as the processing unit 2104.

The system bus 2108 can be any of several types of bus structure that can further interconnect to a memory bus (with or without a memory controller), a peripheral bus, and a local bus using any of a variety of commercially available bus architectures. The system memory 2106 includes ROM 2110 and RAM 2112. A basic input/output system (BIOS) can be stored in a non-volatile memory such as ROM, erasable programmable read only memory (EPROM), EEPROM, which BIOS contains the basic routines that help to transfer information between elements within the computer 2102, such as during startup. The RAM 2112 can also include a high-speed RAM such as static RAM for caching data.

The computer 2102 further includes an internal hard disk drive (HDD) 2114 (e.g., EIDE, SATA), one or more external storage devices 2116 (e.g., a magnetic floppy disk drive (FDD) 2116, a memory stick or flash drive reader, a memory card reader, etc.) and a drive 2120, e.g., such as a solid state drive, an optical disk drive, which can read or write from a disk 2122, such as a CD-ROM disc, a DVD, a BD, etc. Alternatively, where a solid state drive is involved, disk 2122 would not be included, unless separate. While the internal HDD 2114 is illustrated as located within the computer 2102, the internal HDD 2114 can also be configured for external use in a suitable chassis (not shown). Additionally, while not shown in environment 2100, a solid state drive (SSD) could be used in addition to, or in place of, an HDD 2114. The HDD 2114, external storage device(s) 2116 and drive 2120 can be connected to the system bus 2108 by an HDD interface 2124, an external storage interface 2126 and a drive interface 2128, respectively. The interface 2124 for external drive implementations can include at least one or both of Universal Serial Bus (USB) and Institute of Electrical and Electronics Engineers (IEEE) 1394 interface technologies. Other external drive connection technologies are within contemplation of the embodiments described herein.

The drives and their associated computer-readable storage media provide nonvolatile storage of data, data structures, computer-executable instructions, and so forth. For the computer 2102, the drives and storage media accommodate the storage of any data in a suitable digital format. Although the description of computer-readable storage media above refers to respective types of storage devices, it should be appreciated by those skilled in the art that other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 2112, including an operating system 2130, one or more application programs 2132, other program modules 2134 and program data 2136. All or portions of the operating system, applications, modules, or data can also be cached in the RAM 2112. The systems and methods described herein can be implemented utilizing various commercially available operating systems or combinations of operating systems.

Computer 2102 can optionally comprise emulation technologies. For example, a hypervisor (not shown) or other intermediary can emulate a hardware environment for operating system 2130, and the emulated hardware can optionally be different from the hardware illustrated in FIG. 21. In such an embodiment, operating system 2130 can comprise one virtual machine (VM) of multiple VMs hosted at computer 2102. Furthermore, operating system 2130 can provide runtime environments, such as the Java runtime environment or the .NET framework, for applications 2132. Runtime environments are consistent execution environments that allow applications 2132 to run on any operating system that includes the runtime environment. Similarly, operating system 2130 can support containers, and applications 2132 can be in the form of containers, which are lightweight, standalone, executable packages of software that include, e.g., code, runtime, system tools, system libraries and settings for an application.

Further, computer 2102 can be enable with a security module, such as a trusted processing module (TPM). For instance with a TPM, boot components hash next in time boot components, and wait for a match of results to secured values, before loading a next boot component. This process can take place at any layer in the code execution stack of computer 2102, e.g., applied at the application execution level or at the OS kernel level, thereby enabling security at any level of code execution.

A user can enter commands and information into the computer 2102 through one or more wired/wireless input devices, e.g., a keyboard 2138, a touch screen 2140, and a pointing device, such as a mouse 2142. Other input devices (not shown) can include a microphone, an infrared (IR) remote control, a radio frequency (RF) remote control, or other remote control, a joystick, a virtual reality controller or virtual reality headset, a game pad, a stylus pen, an image input device, e.g., camera(s), a gesture sensor input device, a vision movement sensor input device, an emotion or facial detection device, a biometric input device, e.g., fingerprint or iris scanner, or the like. These and other input devices are often connected to the processing unit 2104 through an input device interface 2144 that can be coupled to the system bus 2108, but can be connected by other interfaces, such as a parallel port, an IEEE 1394 serial port, a game port, a USB port, an IR interface, a BLUETOOTH® interface, etc.

A monitor 2146 or other type of display device can be also connected to the system bus 2108 via an interface, such as a video adapter 2148. In addition to the monitor 2146, a computer typically includes other peripheral output devices (not shown), such as speakers, printers, etc.

The computer 2102 can operate in a networked environment using logical connections via wired or wireless communications to one or more remote computers, such as a remote computer(s) 2150. The remote computer(s) 2150 can be a workstation, a server computer, a router, a personal computer, portable computer, microprocessor-based entertainment appliance, a peer device or other common network node, and typically includes many or all of the elements described relative to the computer 2102, although, for purposes of brevity, only a memory/storage device 2152 is illustrated. The logical connections depicted include wired/wireless connectivity to a local area network (LAN) 2154 or larger networks, e.g., a wide area network (WAN) 2156. Such LAN and WAN networking environments are commonplace in offices and companies, and facilitate enterprise-wide computer networks, such as intranets, all of which can connect to a global communications network, e.g., the Internet.

When used in a LAN networking environment, the computer 2102 can be connected to the local network 2154 through a wired or wireless communication network interface or adapter 2158. The adapter 2158 can facilitate wired or wireless communication to the LAN 2154, which can also include a wireless access point (AP) disposed thereon for communicating with the adapter 2158 in a wireless mode.

When used in a WAN networking environment, the computer 2102 can include a modem 2160 or can be connected to a communications server on the WAN 2156 via other means for establishing communications over the WAN 2156, such as by way of the Internet. The modem 2160, which can be internal or external and a wired or wireless device, can be connected to the system bus 2108 via the input device interface 2144. In a networked environment, program modules depicted relative to the computer 2102 or portions thereof, can be stored in the remote memory/storage device 2152. It will be appreciated that the network connections shown are example and other means of establishing a communications link between the computers can be used.

When used in either a LAN or WAN networking environment, the computer 2102 can access cloud storage systems or other network-based storage systems in addition to, or in place of, external storage devices 2116 as described above, such as but not limited to a network virtual machine providing one or more aspects of storage or processing of information. Generally, a connection between the computer 2102 and a cloud storage system can be established over a LAN 2154 or WAN 2156 e.g., by the adapter 2158 or modem 2160, respectively. Upon connecting the computer 2102 to an associated cloud storage system, the external storage interface 2126 can, with the aid of the adapter 2158 or modem 2160, manage storage provided by the cloud storage system as it would other types of external storage. For instance, the external storage interface 2126 can be configured to provide access to cloud storage sources as if those sources were physically connected to the computer 2102.

The computer 2102 can be operable to communicate with any wireless devices or entities operatively disposed in wireless communication, e.g., a printer, scanner, desktop or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH® wireless technologies. Thus, the communication can be a predefined structure as with a conventional network or simply an ad hoc communication between at least two devices.

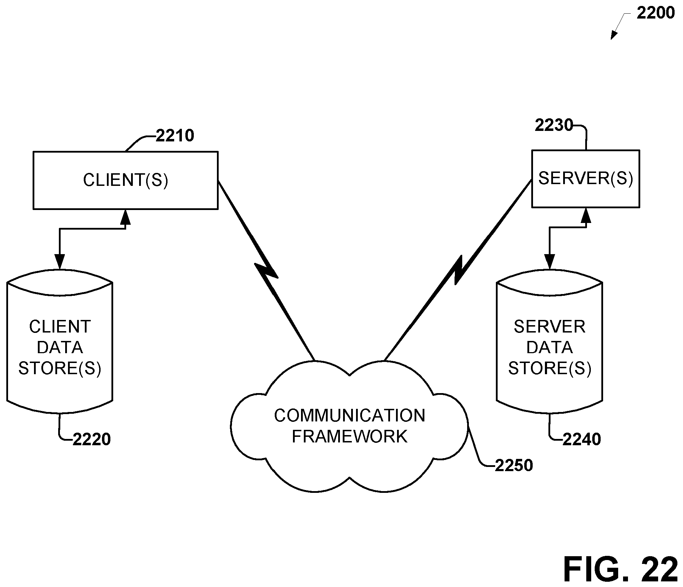

FIG. 22 is a schematic block diagram of a sample computing environment 2200 with which the disclosed subject matter can interact. The sample computing environment 2200 includes one or more client(s) 2210. The client(s) 2210 can be hardware or software (e.g., threads, processes, computing devices). The sample computing environment 2200 also includes one or more server(s) 2230. The server(s) 2230 can also be hardware or software (e.g., threads, processes, computing devices). The servers 2230 can house threads to perform transformations by employing one or more embodiments as described herein, for example. One possible communication between a client 2210 and a server 2230 can be in the form of a data packet adapted to be transmitted between two or more computer processes. The sample computing environment 2200 includes a communication framework 2250 that can be employed to facilitate communications between the client(s) 2210 and the server(s) 2230. The client(s) 2210 are operably connected to one or more client data store(s) 2220 that can be employed to store information local to the client(s) 2210. Similarly, the server(s) 2230 are operably connected to one or more server data store(s) 2240 that can be employed to store information local to the servers 2230.

Various embodiments may be a system, a method, an apparatus or a computer program product at any possible technical detail level of integration. The computer program product can include a computer readable storage medium (or media) having computer readable program instructions thereon for causing a processor to carry out aspects of various embodiments. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium can also include the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network or a wireless network. The network can comprise copper transmission cables, optical transmission fibers, wireless transmission, routers, firewalls, switches, gateway computers or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of various embodiments can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, including a local area network (LAN) or a wide area network (WAN), or the connection can be made to an external computer (for example, through the Internet using an Internet Service Provider). In some embodiments, electronic circuitry including, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform various aspects.

Various aspects are described herein with reference to flowchart illustrations or block diagrams of methods, apparatus (systems), and computer program products according to various embodiments. It will be understood that each block of the flowchart illustrations or block diagrams, and combinations of blocks in the flowchart illustrations or block diagrams, can be implemented by computer readable program instructions. These computer readable program instructions can be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart or block diagram block or blocks. These computer readable program instructions can also be stored in a computer readable storage medium that can direct a computer, a programmable data processing apparatus, or other devices to function in a particular manner, such that the computer readable storage medium having instructions stored therein comprises an article of manufacture including instructions which implement aspects of the function/act specified in the flowchart or block diagram block or blocks. The computer readable program instructions can also be loaded onto a computer, other programmable data processing apparatus, or other device to cause a series of operational acts to be performed on the computer, other programmable apparatus or other device to produce a computer implemented process, such that the instructions which execute on the computer, other programmable apparatus, or other device implement the functions/acts specified in the flowchart or block diagram block or blocks.

The flowcharts and block diagrams in the Figures illustrate the architecture, functionality, and operation of possible implementations of systems, methods, and computer program products according to various embodiments. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams or flowchart illustration, and combinations of blocks in the block diagrams or flowchart illustration, can be implemented by special purpose hardware-based systems that perform the specified functions or acts or carry out combinations of special purpose hardware and computer instructions.

While the subject matter has been described above in the general context of computer-executable instructions of a computer program product that runs on a computer or computers, those skilled in the art will recognize that this disclosure also can or can be implemented in combination with other program modules. Generally, program modules include routines, programs, components, data structures, etc. that perform particular tasks or implement particular abstract data types. Moreover, those skilled in the art will appreciate that various aspects can be practiced with other computer system configurations, including single-processor or multiprocessor computer systems, mini-computing devices, mainframe computers, as well as computers, handheld computing devices (e.g., PDA, phone), microprocessor-based or programmable consumer or industrial electronics, and the like. The illustrated aspects can also be practiced in distributed computing environments in which tasks are performed by remote processing devices that are linked through a communications network. However, some, if not all aspects of this disclosure can be practiced on stand-alone computers. In a distributed computing environment, program modules can be located in both local and remote memory storage devices.

As used in this application, the terms "component," "system," "platform," "interface," and the like, can refer to or can include a computer-related entity or an entity related to an operational machine with one or more specific functionalities. The entities disclosed herein can be either hardware, a combination of hardware and software, software, or software in execution. For example, a component can be, but is not limited to being, a process running on a processor, a processor, an object, an executable, a thread of execution, a program, or a computer. By way of illustration, both an application running on a server and the server can be a component. One or more components can reside within a process or thread of execution and a component can be localized on one computer or distributed between two or more computers. In another example, respective components can execute from various computer readable media having various data structures stored thereon. The components can communicate via local or remote processes such as in accordance with a signal having one or more data packets (e.g., data from one component interacting with another component in a local system, distributed system, or across a network such as the Internet with other systems via the signal). As another example, a component can be an apparatus with specific functionality provided by mechanical parts operated by electric or electronic circuitry, which is operated by a software or firmware application executed by a processor. In such a case, the processor can be internal or external to the apparatus and can execute at least a part of the software or firmware application. As yet another example, a component can be an apparatus that provides specific functionality through electronic components without mechanical parts, wherein the electronic components can include a processor or other means to execute software or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. As used herein, the term "and/or" is intended to have the same meaning as "or." Moreover, articles "a" and "an" as used in the subject specification and annexed drawings should generally be construed to mean "one or more" unless specified otherwise or clear from context to be directed to a singular form. As used herein, the terms "example" or "exemplary" are utilized to mean serving as an example, instance, or illustration. For the avoidance of doubt, the subject matter disclosed herein is not limited by such examples. In addition, any aspect or design described herein as an "example" or "exemplary" is not necessarily to be construed as preferred or advantageous over other aspects or designs, nor is it meant to preclude equivalent exemplary structures and techniques known to those of ordinary skill in the art.

The herein disclosure describes non-limiting examples. For ease of description or explanation, various portions of the herein disclosure utilize the term "each," "every," or "all" when discussing various examples. Such usages of the term "each," "every," or "all" are non-limiting. In other words, when the herein disclosure provides a description that is applied to "each," "every," or "all" of some particular object or component, it should be understood that this is a non-limiting example, and it should be further understood that, in various other examples, it can be the case that such description applies to fewer than "each," "every," or "all" of that particular object or component.

As it is employed in the subject specification, the term "processor" can refer to substantially any computing processing unit or device comprising, but not limited to, single-core processors; single-processors with software multithread execution capability; multi-core processors; multi-core processors with software multithread execution capability; multi-core processors with hardware multithread technology; parallel platforms; and parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, or any combination thereof designed to perform the functions described herein. Further, processors can exploit nano-scale architectures such as, but not limited to, molecular and quantum-dot based transistors, switches and gates, in order to optimize space usage or enhance performance of user equipment. A processor can also be implemented as a combination of computing processing units. In this disclosure, terms such as "store," "storage," "data store," data storage," "database," and substantially any other information storage component relevant to operation and functionality of a component are utilized to refer to "memory components," entities embodied in a "memory," or components comprising a memory. It is to be appreciated that memory or memory components described herein can be either volatile memory or nonvolatile memory, or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory, or nonvolatile random access memory (RAM) (e.g., ferroelectric RAM (FeRAM). Volatile memory can include RAM, which can act as external cache memory, for example. By way of illustration and not limitation, RAM is available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM), and Rambus dynamic RAM (RDRAM). Additionally, the disclosed memory components of systems or computer-implemented methods herein are intended to include, without being limited to including, these and any other suitable types of memory.

What has been described above include mere examples of systems and computer-implemented methods. It is, of course, not possible to describe every conceivable combination of components or computer-implemented methods for purposes of describing this disclosure, but many further combinations and permutations of this disclosure are possible. Furthermore, to the extent that the terms "includes," "has," "possesses," and the like are used in the detailed description, claims, appendices and drawings such terms are intended to be inclusive in a manner similar to the term "comprising" as "comprising" is interpreted when employed as a transitional word in a claim.

The descriptions of the various embodiments have been presented for purposes of illustration, but are not intended to be exhaustive or limited to the embodiments disclosed. Many modifications and variations will be apparent without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

The following paragraphs provide various examples of the embodiments disclosed herein.

EXAMPLE 1: A system can comprise: a processor that can execute computer-executable components stored in a non-transitory computer-readable memory, wherein the computer-executable components can comprise: a matrix computation component that can compute a Markov transition matrix; and a mass spectrum component that can construct a mass spectrum for a molecule based on the Markov transition matrix.

EXAMPLE 2: The system of any preceding example can be implemented wherein the matrix computation component can compute the Markov transition matrix based on respective reaction probabilities associated with fragmentation of the molecule into a plurality of ion fragments, and wherein the computer-executable components can further comprise: a probability determination component that can transform, based on the Markov transition matrix, the respective reaction probabilities into respective fragment probabilities associated with the fragmentation of the molecule.

EXAMPLE 3: The system of any preceding example can be implemented wherein the Markov transition matrix represents a transition of the molecule from an unfragmented state to a fragmented state, and wherein the probability determination component can further: compute, based on the Markov transition matrix, a desired state within the fragmentation of the molecule via a random walk process; and model, based on the random walk process, the fragmentation of the molecule as a Markov process.

EXAMPLE 4: The system of any preceding example can be implemented wherein a fragment probability of the respective fragment probabilities represents a probability of an ion fragment being generated during the fragmentation, and wherein the computer-executable components can further comprise: a peak intensity computation component that can compute a sum of fragment probabilities of respective ion fragments having identical masses.

EXAMPLE 5: The system of any preceding example can be implemented wherein the computer-executable components can further comprise: a display component that can display the sum of fragment probabilities as a peak on a spectrogram.

EXAMPLE 6: The system of any preceding example can be implemented wherein the respective reaction probabilities can be generated by an optimization algorithm based on a neural network, and wherein the respective reaction probabilities can be defined within a fragmentation graph that can be accessible to the mass spectrum component.

EXAMPLE 7: The system of any preceding example can be implemented wherein construction of the mass spectrum based on the Markov transition matrix can reduce a computational load and increase a computational speed involved in the construction.

EXAMPLE 8: The system of any preceding example can be implemented wherein the computer-executable components can further comprise: a spectral database component that can generate a spectral database based on the mass spectrum, wherein the spectral database can be employable for compound identification.

EXAMPLE 9: The system of any preceding example can be implemented wherein a number of steps of the fragmentation of the molecule is limited based on a target simulated energy.

In various embodiments, any combination or combinations of examples 1 - 9 can be implemented.

EXAMPLE 10: A computer-implemented method can comprise: computing, by a device operatively coupled to a processor, a Markov transition matrix; and constructing, by the device, a mass spectrum for a molecule based on the Markov transition matrix.

EXAMPLE 11: The computer-implemented method of any preceding example can be implemented wherein the computer-implemented method can further comprise: the computing, by the device, the Markov transition matrix based on respective reaction probabilities associated with fragmentation of the molecule into a plurality of ion fragments; and transforming, by the device, based on the Markov transition matrix, the respective reaction probabilities into respective fragment probabilities associated with the fragmentation of the molecule.

EXAMPLE 12: The computer-implemented method of any preceding example can be implemented wherein the Markov transition matrix can represent a transition of the molecule from an unfragmented state to a fragmented state, and wherein the computer-implemented method can further comprise: computing, by the device, based on the Markov transition matrix, a desired state within the fragmentation of the molecule via a random walk process; and modeling, by the device, based on the random walk process, the fragmentation of the molecule as a Markov process.

EXAMPLE 13: The computer-implemented method of any preceding example can be implemented wherein a fragment probability of the respective fragment probabilities represents a probability of an ion fragment being generated during the fragmentation, and wherein the computer-implemented method can further comprise: computing, by the device, a sum of fragment probabilities of respective ion fragments having identical masses.

EXAMPLE 14: The computer-implemented method of any preceding example can be implemented wherein the constructing can further comprise: displaying, by the device, the sum of fragment probabilities as a peak on a spectrogram.

EXAMPLE 15: The computer-implemented method of any preceding example can be implemented wherein the respective reaction probabilities can be generated by an optimization algorithm based on a neural network, and wherein the respective reaction probabilities can be defined within a fragmentation graph that is accessible to the device.

EXAMPLE 16: The computer-implemented method of any preceding example can be implemented wherein construction of the mass spectrum based on the Markov transition matrix can reduce a computational load and increase a computational speed involved in the construction.

EXAMPLE 17: The computer-implemented method of any preceding example can be implemented wherein the computer-implemented method can further comprise: generating, by the device, a spectral database based on the mass spectrum, wherein the spectral database can be employable for compound identification.

In various embodiments, any combination or combinations of examples 10 - 17 can be implemented.

EXAMPLE 18: A computer program product for constructing in silico mass spectra of compounds can comprise a non-transitory computer-readable memory having program instructions embodied therewith. The program instructions can be executable by a processor to cause the processor to: compute a Markov transition matrix; and construct a mass spectrum for a molecule based on the Markov transition matrix.

EXAMPLE 19: The computer program product of any preceding example can be implemented wherein the program instructions can be further executable by the processor to cause the processor to: compute the Markov transition matrix based on respective reaction probabilities associated with fragmentation of the molecule into a plurality of ion fragments; and transform, based on the Markov transition matrix, the respective reaction probabilities into respective fragment probabilities associated with the fragmentation of the molecule.

EXAMPLE 20: The computer program product of any preceding example can be implemented wherein the Markov transition matrix represents a transition of the molecule from an unfragmented state to a fragmented state, and wherein the program instructions can be further executable by the processor to cause the processor to: compute, based on the Markov transition matrix, a desired state within the fragmentation of the molecule via a random walk process; and model, based on the random walk process, the fragmentation of the molecule as a Markov process.

In various embodiments, any combination or combinations of examples 18 - 20 can be implemented.

In various embodiments, any combination or combinations of examples 1 - 20 can be implemented.

**Claims**

1. A computer-implemented method, comprising:

   computing, by a device operatively coupled to a processor, a Markov transition matrix; and
   constructing, by the device, a mass spectrum for a molecule based on the Markov transition matrix.

2. The computer-implemented method of claim 1, wherein the computing, by the device, the Markov transition matrix is based on respective reaction probabilities associated with fragmentation of the molecule into a plurality of ion fragments, and wherein the computer-implemented method further comprises:
   transforming, by the device, based on the Markov transition matrix, the respective reaction probabilities into respective fragment probabilities associated with the fragmentation of the molecule.

3. The computer-implemented method of claim 2, wherein the Markov transition matrix represents a transition of the molecule from an unfragmented state to a fragmented state, and wherein the computer-implemented method further comprises:

   computing, by the device, based on the Markov transition matrix, a desired state within the fragmentation of the molecule via a random walk process; and
   modeling, by the device, based on the random walk process, the fragmentation of the molecule as a Markov process.

4. The computer-implemented method of claim 2, wherein a fragment probability of the respective fragment probabilities represents a probability of an ion fragment being generated during the fragmentation, and wherein the computer-implemented method further comprises:
   computing, by the device, a sum of fragment probabilities of respective ion fragments having identical masses.

5. The computer-implemented method of claim 4, wherein the constructing further comprises:
   displaying, by the device, the sum of fragment probabilities as a peak on a spectrogram.

6. The computer-implemented method of claim 2, wherein the respective reaction probabilities are generated by an optimization algorithm based on a neural network, and wherein the respective reaction probabilities are defined within a fragmentation graph that is accessible to the device.

7. The computer-implemented method of any preceding claim, wherein construction of the mass spectrum based on the Markov transition matrix reduces a computational load and increases a computational speed involved in the construction.

8. The computer-implemented method of any preceding claim, further comprising:
   generating, by the device, a spectral database based on the mass spectrum, wherein the spectral database is employable for compound identification.

9. A computer program for constructing in silico mass spectra of compounds, the computer program comprising program instructions executable by a processor to cause the processor to carry out the method steps of any preceding claim.

10. A computer program product comprising a non transitory computer readable memory upon which is stored the computer program of claim 9.

**11.** A system comprising a non transitory computer readable memory upon which is stored the computer program of claim 9, and a processor configured to execute the program instructions of that computer program.

SCIENTIFIC INSTRUMENT SUPPORT
MODULE **102**

FIRST (MATRIX
COMPUTATION) LOGIC **104**

SECOND (MASS SPECTRUM
CONSTRUCTION) LOGIC **106**

**FIG. 1**

200

PERFORM FIRST OPERATIONS COMPUTING, BY A DEVICE OPERATIVELY COUPLED TO A PROCESSOR, A MARKOV TRANSITION MATRIX — 202

PERFORM SECOND OPERATIONS CONSTRUCTING, BY THE DEVICE, A MASS SPECTRUM FOR A MOLECULE BASED ON THE MARKOV TRANSITION MATRIX — 204

# FIG. 2

EP 4 708 303 A1

**FIG. 3**

FIG. 4

EP 4 708 303 A1

SYSTEM **302**

SOFTWARE **304**

MATRIX COMPUTATION COMPONENT **310**

MASS SPECTRUM COMPONENT **312**

SPECTRAL DATABASE COMPONENT **502**

PROCESSOR **306**

307

MEMORY **308**

SYSTEM **318**

FRAGMENTATION GRAPH **322**

FRAGMENTATION GRAPH **324**

SPECTROGRAM **408**

MASS SPECTRUM **326**

SPECTRAL DATABASE **504**

**FIG. 5**

EP 4 708 303 A1

600

COMPOUND IDENTIFICATION
SYSTEM **604**

ACCESS
COMPONENT **608**

INPUT DATA **602** →

→ COMPOUND
INFORMATION **606**

MASS SPECTRA
COMPARISON
COMPONENT **610**

NON-LIMITING
SYSTEM **300**

**FIG. 6**

**FIG. 7**

EP 4 708 303 A1

EP 4 708 303 A1

FIG. 8A

FIG. 8B

EP 4 708 303 A1

EP 4 708 303 A1

**FIG. 8C**

**FIG. 9**

EP 4 708 303 A1

FIG. 10

EP 4 708 303 A1

FIG. 11

FIG. 12

FIG. 13A

FIG. 13B

1400

1410

1420

FIG. 14

**FIG. 15A**

1502

#0 RT: 0.00 NL: 8.78E-1

Relative Abundance

91.0542

70.0287    148.0393    206.0812    268.1332
42.0338  107.0491    178.0863  229.0859              314.1387

m/z

1512

#0 RT: 0.00 NL: 9.21E-1

Relative Abundance

91.0542

30.0338      116.0495      200.1070            315.1492
    80.0495        159.0553    226.0863  288.1383    358.1550

m/z

1522

#0 RT: 0.00 NL: 6.79E-1

Relative Abundance

105.0699

188.1434
84.0808      159.1168              253.1699    323.2118
43.0542    119.0855    203.1543    281.2012    351.2431

m/z

FIG. 15B

FIG. 16

1700

COMPUTING, BY A DEVICE OPERATIVELY COUPLED TO A PROCESSOR, A MARKOV TRANSITION MATRIX — 1702

CONSTRUCTING, BY THE DEVICE, A MASS SPECTRUM FOR A MOLECULE BASED ON THE MARKOV TRANSITION MATRIX — 1704

EMPLOYING, BY THE DEVICE, THE MASS TO GENERATE A SPECTRAL DATABASE FOR COMPOUND IDENTIFICATION — 1706

ACCESSING, BY THE DEVICE, INFORMATION ABOUT AN UNKNOWN COMPOUND TO BE IDENTIFIED — 1708

1714 — NO

DOES A MASS SPECTRUM FOR THE UNKNOWN COMPOUND EXIST IN THE SPECTRAL DATABASE? — 1710

YES

IDENTIFYING, BY THE DEVICE, THE UNKNOWN COMPOUND — 1712

**FIG. 17**

1800

DATA DISPLAY REGION 1802

DATA ANALYSIS REGION 1804

SCIENTIFIC
INSTRUMENT
CONTROL
REGION 1806

SETTINGS
REGION 1808

# FIG. 18

COMPUTING DEVICE 1900

| PROCESSING DEVICE 1902 | BATTERY/POWER 1908 |
| STORAGE DEVICE 1904 | DISPLAY DEVICE 1910 |
| INTERFACE DEVICE 1906 | OTHER I/O DEVICES 1912 |

**FIG. 19**

**2000**

REMOTE
COMPUTING
DEVICE

PROCESSING
DEVICE
2002

STORAGE
DEVICE
2004

INTERFACE
DEVICE
2006

SCIENTIFIC
INSTRUMENT
2010

PROCESSING
DEVICE
2002

STORAGE
DEVICE
2004

INTERFACE
DEVICE
2006

SERVICE LOCAL
COMPUTING
DEVICE

PROCESSING
DEVICE
2002

STORAGE
DEVICE
2004

INTERFACE
DEVICE
2006

USER LOCAL
COMPUTING
DEVICE

PROCESSING
DEVICE
2002

STORAGE
DEVICE
2004

INTERFACE
DEVICE
2006

2008

2008

2008

2008

2008

2008

2012

**FIG. 20**

FIG. 21

2200

2210

CLIENT(S)

2230

SERVER(S)

CLIENT
DATA
STORE(S)

2220

COMMUNICATION
FRAMEWORK

2250

SERVER
DATA
STORE(S)

2240

# FIG. 22

Europäisches
Patentamt
European
Patent Office
Office européen
des brevets

## EUROPEAN SEARCH REPORT

**Application Number**

EP 25 19 9201

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ALLEN FELICITY ET AL: "Competitive fragmentation modeling of ESI-MS/MS spectra for putative metabolite identification", METABOLOMICS, SPRINGER US, NEW YORK, vol. 11, no. 1, 5 June 2014 (2014-06-05), pages 98-110, XP035425021, ISSN: 1573-3882, DOI: 10.1007/S11306-014-0676-4 [retrieved on 2014-06-05] | 1-5,7-11 | INV. G16C20/20 G16C20/70 ADD. G16B40/10 |
| Y | * figures 1,2 * | 6 | |
| X | FRANCESCO F RUSSO ET AL: "Machine learning methods for compound annotation in non-targeted mass spectrometry-A brief overview of fingerprinting, in silico fragmentation and de novo methods", RAPID COMMUNICATIONS IN MASS SPECTROMETRY, JOHN WILEY & SONS, GB, vol. 38, no. 20, 24 August 2024 (2024-08-24), page n/a, XP072697021, ISSN: 0951-4198, DOI: 10.1002/RCM.9876 * Section 3 "In silico fragmentation" * | 1,9-11 | |
| Y | GOLDMAN SAMUEL ET AL: "Generating Molecular Fragmentation Graphs with Autoregressive Neural Networks", ANALYTICAL CHEMISTRY, vol. 96, no. 8, 13 February 2024 (2024-02-13), pages 3419-3428, XP093354996, ISSN: 0003-2700, DOI: 10.1021/acs.analchem.3c04654 Retrieved from the Internet: URL:https://pubs.acs.org/doi/pdf/10.1021/acs.analchem.3c04654> * figure 2 * | 6 | TECHNICAL FIELDS SEARCHED (IPC) G16C G16B |

The present search report has been drawn up for all claims

| Place of search | Date of completion of the search | Examiner |
|---|---|---|
| Munich | 19 January 2026 | Heidrich, Alexander |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

**Patent documents cited in the description**

- WO 63689981 A